(19) Europäisches Patentamt
European Patent Office
Office européen des brevets

(11) EP 0 439 548 B1

(12) **EUROPEAN PATENT SPECIFICATION**

(45) Date of publication and mention
of the grant of the patent:
**27.03.1996 Bulletin 1996/13**

(21) Application number: **89912747.6**

(22) Date of filing: **17.10.1989**

(51) Int Cl.6: **G01N 33/49**, G01N 33/50,
G01N 33/574
// G01N33/52

(86) International application number:
**PCT/US89/04658**

(87) International publication number:
**WO 90/04785 (03.05.1990 Gazette 1990/10)**

(54) **PROVISION OF DENSITY SPECIFIC BLOOD CELLS FOR THE STRUCTUREDNESS OF THE CYTOPLASMIC MATRIX (SCM) TEST**

PRÄPARAT VON BLUTZELLEN SPEZIFISCHER DICHTE FÜR DEN SCM-TEST

CELLULES SANGUINES SPECIFIQUES A LA DENSITE POUR LA STRUCTURATION DU TEST DE LA MATRICE CYTOPLASMIQUE (SCM)

(84) Designated Contracting States:
**AT BE CH DE FR GB IT LI LU NL SE**

(30) Priority: **21.10.1988 US 260928**

(43) Date of publication of application:
**07.08.1991 Bulletin 1991/32**

(73) Proprietors:
- **CERCEK, Boris**
  **Yorba Linda, CA 92686 (US)**
- **CERCEK, Lea**
  **Yorba Linda, CA 92686 (US)**

(72) Inventors:
- **CERCEK, Boris**
  **Yorba Linda, CA 92686 (US)**

- **CERCEK, Lea**
  **Yorba Linda, CA 92686 (US)**

(74) Representative: **Goldin, Douglas Michael et al**
**J.A. KEMP & CO.**
**14, South Square**
**Gray's Inn**
**London WC1R 5LX (GB)**

(56) References cited:
**WO-A-87/05393          DE-A- 3 222 452**

- **BRITISH JOURNAL OF CANCER, Vol. 38, 1978
  L Cercek et al: pp. 163-165**
- **Methods in Enzymology, Vol. 108, 1984 K
  Shorman: pp. 102-117**

## Description

BACKGROUND

This application discloses improved methods for isolating SCM-responding lymphocytes for the performance of the SCM test useful in the detection of cancer and other diseases and conditions.

Many diseases occurring in humans and animals can be detected by the presence of foreign substances, particularly in the blood, which are specifically associated with a disease or condition. Tests for antigens or other such substances produced as a result of such diseases show great promise as a diagnostic tool for the early detection and treatment of the particular disease that produced the antigen or other substance. Procedures for the detection of such substances must be reliable, reproducible, and sensitive in order to constitute a practical diagnostic procedure for health care providers. In addition any such procedure should be able to be carried out by persons of ordinary skill and training in laboratory procedure, and should be relatively fast and inexpensive.

For example, in the treatment of the various malignancies that afflict humans and animals, referred to generally as cancer, it is recognized that early detection is a key to effective treatment, especially as many therapeutic procedures are effective only in relatively early stages of the disease. In fact, virtually all known cancer treatments are not only more effective, but safer, when administered in early stages of cancer. Far too many cases of cancer are only discovered too late for effective treatment.

Accordingly, there is a great need for rapid, easy-to-perform, and reliable tests which can diagnose cancer at early stages. In this connection, new tests and procedures are being developed to effect early diagnosis of the cancer.

We have developed and reported one such test for the early detection of cancer in L. Cercek, B. Cercek, and C.I.V. Franklin, "Biophysical Differentiation Between Lymphocytes from Healthy Donors, Patients with Malignant Disease and Other Disorders," Brit. J. Cancer 29, 345-352 (1974) and L. Cercek and B. Cercek, "Application of the Phenomenon of Changes in the Structuredness of Cytoplasmic Matrix (SCM) in the Diagnosis of Malignant Disorders: a Review", Europ. J. Cancer 13, 903-915 (1977), which are incorporated herein by this reference.

Our basic SCM test includes the steps of:

(1) challenging a selected subpopulation of lymphocytes from a donor with a challenging agent such as a mitogen or an antigen associated with a condition or disease, such as cancer; and
(2) determining the change in structuredness of the cytoplasmic matrix (SCM) of the challenged lymphocytes, typically using fluorescence polarization.

SCM reflects the forces of interaction between macromolecules of the cell and other cellular components such as water molecules, ions, adenosine triphosphate, and cyclic adenosine phosphate. Perturbations of these interactions result in changes in SCM.

When applied to cancer, our SCM test is based on the phenomenon that the internal structure of a selected subpopulation of the lymphocytes from a healthy individual is altered when challenged by a mitogen such as phytohaemagglutinin, but is not altered by other selected challenging agents, such as cancer basic protein (CaBP) and/or antigens derived from specific malignant tumors such as tumor associated antigens (TAAs). Contrarily, the equivalent subpopulation of lymphocytes from an individual with cancer responds oppositely. In other words the same subpopulation of lymphocytes from cancer patients does not respond in the SCM test when challenged by a mitogen, but does respond when challenged by a number of cancer-associated antigens. When TAAs derived from specific malignant tumors are used as challenging agents, only lymphocytes from individuals with the same type of tumor from which the TAA had been isolated will respond in the SCM test.

The changes seen in SCM are believed to reflect changes in the internal structure of the lymphocyte as the lymphocyte is activated for synthesis. Similar changes can occur in living cells other than lymphocytes during the cell cycle and growth of the cells. Such changes can also be evoked by various external agents, such as ionizing radiation, mechanical forces, chemicals, growth inhibiting and stimulating agents, etc. These changes can be conveniently monitored with a specially adapted technique of fluorescein fluorescence polarization, as we have published in numerous articles, including L. Cercek and B. Cercek, "Studies on the Structuredness of Cytoplasm and Rates of Enzymatic Hydrolysis in Growing Yeast Cells. I. Changes Induced by Ionizing Radiation," Int. J. Radiat. Biol. 21, 445-453 (1972); L. Cercek and B. Cercek, "Studies on the Structuredness of Cytoplasm and Rates of Enzymatic Hydrolysis in Growing Yeast Cells. II. Changes Induced by Ultra-Violet Light," Int. J. Radiat. Biol. 22, 539-544 (1972); L. Cercek and B. Cercek, "Relationship Between Changes in the Structuredness of Cytoplasm and Rate Constants for the Hydrolysis of FDA in Saccharomyces cerevisiae," Biophysik 9, 109-112 (1973); L. Cercek, B. Cercek, and C.H. Ockey, "Structuredness of the Cytoplasmic Matrix and Michaelis-Menten Constants for the Hydrolysis of FDA During the Cell Cycle in Chinese Hamster Ovary Cells," Biophysik 10, 187-194 (1973); B.I. Lord, L. Cercek, B. Cercek, G.P. Shah, T.M. Dexter and L.G. Lajtha, "Inhibitors of Haemopoietic Cell Proliferation: Specificity of Action Within the Haemopoietic System," Brit. J. Cancer 29, 168-175 (1974); L. Cercek and B. Cercek, "Involvement of Cyclic-AMP in Changes of the Structuredness of Cytoplasmic Matrix," Radiat. & Environ. Biophys. 11, 209-212 (1974); L. Cercek, P. Milenkovic, B. Cercek, & L.G. Lajtha, "Induction of PHA Response in Mouse Bone Marrow Cells by Thymic Extracts as Studied by Changes in the Structuredness of Cytoplasmic Matrix," Immunology 29, 885-891 (1975); L. Cercek and B. Cercek, "Effects of Osmomolarity, Calcium and Magnesium Ions on the Structuredness of Cytoplasmic Matrix (SCM)," Radiat. & Environ. Biophys. 13, 9-12 (1976); L. Cercek & B. Cercek, "Changes in the Structuredness of Cytoplasmic Matrix (SCM) Induced in Mixed Lymphocyte Reactions," Radiat. & Environ. Biophys. 13, 71-74 (1976); L. Cercek, B. Cercek, & C.H. Ockey, "Fluorescein Excitation and Emission Polarization Spectra in Living Cells: Changes During the Cell Cycle," Biophys. J. 23, 395-405 (1978); L. Cercek and B. Cercek, "Effect of Osmolality and Density of Gradients on the Isolation of SCM-Responding Lymphocytes," Brit. J. Cancer 38, 163-165 (1978); L. Cercek and B. Cercek, "Involvement of Mitochondria in Changes of Fluorescein Excitation and Emission Polarization Spectra in Living Cells," Biophys. J. 28, 403-412 (1979); L. Cercek, B. Cercek, and B.I. Lord, "The Effect of Specific Growth Inhibitors on Fluorescein Fluorescence Polarization Spectra in Haemopoietic Cells," Brit. J. Cancer, 44, 749-752 (1981); and L. Cercek and B. Cercek, "Effects of Ascorbate Ions on Intracellular Fluorescein Emission Polarization Spectra in Cancer and Normal Proliferating Cells," Cancer Detection and Prevention 10, 1-20 (1987), all of which are incorporated herein by this reference.

The usefulness of this SCM test for the detection of cancer has been documented in numerous articles. Articles from our laboratory include: L. Cercek, B. Cercek, and J.V. Garrett, "Biophysical Differentiation Between Normal Human and Chronic Lymphocytic Leukaemia Lymphocytes," in Lymphpocyte Recognition and Effector Mechanisms (K. Lindahl-Kiessling and D. Osoba eds., New York, Academic Press, 1974), pp. 553-558; L. Cercek, B. Cercek and C.I.V. Franklin, "Biophysical Differentiation between Lymphocytes from Healthy Donors, Patients with Malignant Disease and Other Disorders," Brit. J. Cancer 29, 345-352 (1974); L. Cercek and B.Cercek, "Changes in the SCM Response Ratio ($RR_{SCM}$) After Surgical Removal of Malignant Tissue," Brit. J. Cancer 31, 250-251 (1975); L. Cercek and B. Cercek, "Apparent Tumour Specificity with the SCM Test," Brit. J. Cancer 31, 252-253 (1975); L. Cercek and B. Cercek, "Changes in the Structuredness of Cytoplasmic Matrix of Lymphocytes as a Diagnostic and Prognostic Test for Cancer," in Cell Biology and Tumour Immunology, Excerpta Medica International Congress Series No. 349, Proceedings of the XI International Cancer Congress, Florence, 1974 (Amsterdam, Excerpta Medica, 1974), vol. 1, pp. 318-323; L. Cercek and B. Cercek, "Application of the Phenomenon of Changes in the Structuredness of Cytoplasmic Matrix (SCM) in the Diagnosis of Malignant Disorders: a Review," Europ. J. Cancer 13, 903-915 (1977); L. Cercek and B. Cercek, "Detection of Malignant Diseases by Changes in the Structuredness of Cytoplasmic Matrix of Lymphocytes Induced by Phytohaemagglutinin and Cancer Basic Proteins," in Tumour Markers, Determination and Clinical Role: Proceedings of the Sixth Tenovus Workshop, Cardiff, April 1977 (K. Griffith, A.M. Neville, and C.G. Pierrepoint, eds., Cardiff, Alpha Omega Publishing Co., 1978), pp. 215-226; and L. Cercek and B. Cercek, "Changes in SCM-Responses of Lymphocytes in Mice After Implantation with Ehrlich Ascites Cells," Europ. J. Cancer 17, 167-171 (1981), all of which are incorporated herein by this reference.

The usefulness of the SCM test has been confirmed in articles from other laboratories, including F. Takaku, K. Yamanaka, and Y. Hashimoto, "Usefulness of the SCM Test in the Diagnosis of Gastric Cancer," Brit. J. Cancer 36, 810-813 (1977); H. Kreutzmann, T.M. Fliedner, H. J. Galla, and E. Sackmann, "Fluorescence-Polarization Changes in Mononuclear Blood Leucocytes After PHA Incubation: Differences in Cells from Patients with and Without Neoplasia," Brit. J. Cancer 37, 797-805 (1978); Y. Hashimoto, T. Yamanaka, and F. Takaku, "Differentiation Between Patients with Malignant Diseases and Non-Malignant Diseases or Healthy Donors by Changes of Fluorescence Polarization in the Cytoplasm of Circulating Lymphocytes," Gann 69, 145-149 (1978); J.A.V. Pritchard and W.H. Sutherland, "Lymphocyte Response to Antigen Stimulation as Measured by Fluorescence Polarization (SCM-Test)," Brit. J. Cancer 38, 339-343 (1978); J.A.V. Pritchard, J.E. Seaman, I.H. Evans, K. W. James, W.H. Sutherland, T.J. Deeley, I.J. Kerby, I.C.M. Patterson, and B.H. Davies, "Cancer-Specific Density Changes in Lymphocytes Following Stimulation with Phytohaemagglutinin," Lancet 11, 1275-1277 (Dec. 16, 1978); H. Orjasaeter, G. Jordfald, and I. Svendsen, "Response of T-Lymphocytes to Phytohaemagglutinin (PHA) and to Cancer-Tissue-Associated Antigens, Measured by the Intracellular Fluorescence Polarization Technique (SCM Test)," Brit. J. Cancer 40, 628-633 (1979); N.D. Schnuda, "Evaluation of Fluorescence Polarization of Human Blood Lymphocytes (SCM Test) in the Diagnosis of Cancer," Cancer 46, 1164-1173 (1980); J.A.V. Pritchard, W.H. Sutherland, J.E. Siddall, A.J. Bater, I.J. Kerby, T.J. Deeley, G. Griffith, R. Sinclair, B.H. Davies, A. Rimmer, & D.J.T. Webster, "A Clinical Assessment of Fluorescence Polarisation Changes in Lymphocytes Stimulated by Phytohaemagglutinin (PHA) in Malignant and Benign Disease," Europ. J. Cancer, Clin. Oncol. 18, 651-659 (1982); G. R. Hocking, J.M. Rolland, R.C. Nairn, E. Pihl, A.M. Cuthbertson, E.S.R. Hughes, and W.R. Johnson, "Lymphocyte Fluorescence Polarization Changes After Phytohaemagglutinin Stimulation in the Diagnosis of Colorectal Carcinoma," J. National Cancer Inst. 68, 579-583 (1982); M. Deutsch and A. Weinreb, "Validation of the SCM-Test for the Diagnosis of Cancer," Eur. J. Cancer, Clin. Oncol. 19, 187-193 (1983); S. Chaitchik, O. Asher, M. Deutsch, and A. Weinreb, "Tumour Specificity of the SCM Test for Cancer Diagnosis," Europ. J. cancer, Clin. Oncol. 21, 1165-1170 (1985); and J. Matsumoto, T. Tenzaki and T. Ishiguro, "Clinical Evaluation of Fluorescein Polarization of Peripheral Lymphocytes (SCM Test) in the Diagnosis of Cancer," J. Japan Soc. Cancer Ther. 20, 728-734 (1985), all of which are incorporated herein by this reference.

As reported, the SCM test indicates usefulness both as a screening test for the general detection of malignancies and as a test to diagnose specific types of malignancies. This has been confirmed by blind clinical tests and corroborated by other investigators.

The SCM test can be applied to detection of diseases and conditions other than cancer, such as viral and bacterial infections, including the detection of the AIDS virus, determination of allergic reactions, tissue typing, and monitoring of allograft rejections such as organ transplant rejection based on the SCM responses in mixed lymphocyte reactions, as disclosed in the 1976 Radiation and Environmental Biophysics article by L. Cercek and B. Cercek. The presence of other antigen-producing diseases and bodily conditions does not interfere with the SCM test; a patient afflicted with more than one type of antigen-producing disease can be tested for a multiplicity of such diseases simply by running separate tests using for each test an antigen derived from each separate disease or condition being tested for.

When fluorescence polarization is used to determine changes of SCM, such changes are seen as a decrease in the fluorescence polarization of the cells when polarized light is used to excite an intrinsic fluor generated intracellularly by the hydrolysis of a non-fluorescent compound which has been absorbed by the lymphocytes. The fluor typically is fluorescein and the non-fluorogenic compound is typically fluorescein diacetate (FDA). The FDA serves as a fluorogenic agent precursor. An extrinsic fluor is used because the intrinsic fluorescence of cellular components is too small to give meaningful results in this test. Therefore, all references to fluorescence polarization values herein are references to fluorescence polarization values obtained with an extrinsic fluor, preferably one generated by enzymatic hydrolysis from a non-fluorogenic compound added to and absorbed by the cells. Typically, excitation of the fluor occurs with light at 470 nm and the intracellular fluorescein fluorescence polarization peak occurs at 510 nm. Excitation at 442 nm can also be used, in which case the intracellular fluorescein polarization peak occurs at 527 nm.

Intracellular fluorescence polarization is a measure of resistance to rotational relaxation of fluorescein molecules; the greater the intracellular resistance to rotation, the greater the measured fluorescence polarization. As seen in the SCM test, a decrease in fluorescence polarization is believed to result mainly from changes in the conformation of the mitochondria, the energy-producing organelles of the cell. Changes in the mitochondria are believed to result from the contractions of the cristae or inner folds of the mitochondrial membrane, as stated in L. Cercek & B. Cercek, "Involvement of Mitochondria in Changes of Fluorescein Excitation and Emission Polarization Spectra in Living Cells," Biophys. J., 28, 403-412 (1979).

Immunologically the SCM-responding lymphocytes are T-cell mononuclear leukocytes. Although not fully understood, it is believed that SCM-responding lymphocytes are involved in the recognition of antigens that are circulating in the blood stream. This recognition of antigens triggers the body's immune system. Accordingly, these cells become primed to recognize foreign substances, such as antigens, produced by the disease or condition affecting the body. Not all lymphocytes found in the bloodstream, however, are SCM-responding; only selected sub-populations of cells having specific and narrowly defined buoyant densities-are SCM-responding.

In order to carry out the SCM test successfully, preferably the selected sub-population of SCM-responding lymphocytes must be isolated from other blood components, including all non-SCM-responding lymphocytes. In our prior work, as described in the 1977 European Journal of Cancer article, we isolated SCM-responding lymphocytes on Ficoll $_{(TM)}$-Triosil$_{(TM)}$ gradients after removal of the phagocytic cells from the blood sample by treatment with iron powder or carbonyl-iron powder. The SCM-responding lymphocytes isolated were those that floated on top of a Ficoll-Triosil gradient with a density of 1.081 g/cm$^3$ and an osmolality of 0.320 Osm/kg, while non-responding cells banded inside the gradient. The density figure of 1.081 g/cm$^3$ refers to the density of the gradient solution itself; the actual buoyant density of the SCM-responding cells themselves was not determined, but must be somewhat less than 1.081 g/cm$^3$. The SCM-responding cells were collected from the gradient with a Pasteur pipette, taking great care to avoid the collection of blood components separated above the narrow band of SCM-responding lymphocytes or cells that separated in the density gradient solution below the narrow band. This procedure requires considerable practice and manual dexterity to carry out properly. Additionally, the conditions for isolation and separation of the SCM-responding lymphocytes, such as the temperature of the blood sample and the density gradient solution, and the density, osmolality, and pH of the solution must be rigorously controlled. For example, the temperature should be controlled to ±0.2°C. Failure to control any of these isolation and separation conditions can result either in loss of some of the desired SCM-responding cells or the isolation of some of the non-responding cells along with the desired SCM-responding cells, as stated in L. Cercek & B. Cercek, "Effect of Osmolality and Density of Gradients on the Isolation of SCM-Responding Lymphocytes," Brit. J. Cancer 38, 163-165 (1978). Such errors can result in loss of sensitivity of the SCM test. Indeed, a number of other laboratories attempting to corroborate our work have failed to obtain reliable and reproducible results because of poor or unskilled technique in the separation of the SCM-responding lymphocytes.

This need for precise separation of the SCM-responding lymphocytes became even more important when it was realized that there exists more than one subpopulation of SCM-responding cells, and that these subpopulations yield markedly different responses in the SCM test. The existence of such subpopulations was reported in the 1978 Lancet article by Pritchard et al., in the 1982 European Journal of Cancer Clinical Oncology article by Pritchard et al., and in J.M. Rolland, R.C. Nairn, A.P. Nind, and E. Pihl, "Significance of Lymphocyte Fluorescence Polarization Changes After Phytohaemagglutinin Stimulation in Cancer and Non-cancer Conditions," J. National Cancer Institute 72, 267-273 (1984). These studies revealed that besides the subset of SCM-responding cells that had been isolated in previous SCM studies, there exists an additional subset of SCM-responding cells with a buoyant density different from that of the first set. Unlike the SCM-responding cells previously studied, this additional subset of cells only responded to PHA, and did so only when isolated from cancer patients. It did not respond to any cancer-associated antigens such as CaBP, whether or not isolated from patients with malignancies, and failed to respond to PHA when isolated from patients without cancer. In summary, the response of this subset of cells to PHA is exactly the opposite of that of the first subset. Clearly, it is necessary to separate these two subsets of cells in performing the SCM assay. Otherwise, the results are impossible to interpret, especially when PHA is used to stimulate the SCM-responding cells.

International Publication No. W087/05393 discloses a method for the detection of certain diseases and body conditions based upon the response to antigens associated with the disease or condition tested for by lymphocytes having buoyant densities of $1.0590 g/cm^3$ to $1.0670 g/cm^3$ and densities of $1.0690 g/cm^3$ to $1.0730 g/cm^3$ in a continuous density gradient solution having an osmolality of 0.315 to 0.320 osm/kg at a temperature of 20°C.

The article in the British Journal of Cancer, Volume 38, 1978, by L. CERCEK ET AL entitled "Effects of Osmolality and Density of Gradients on the Isolation of SCM-responding Lymphocytes" at pages 163 to 165 discloses an experimental procedure for determining the exact temperature at which the density gradient of a sample of blood following centrifugation will have the density required for the isolation of the enriched SCM-responding population of lymphocytes.

The article in Methods in Enzymology, Volume 108, 1984 by K. SHORTMAN entitled "Analytical and Preparative Equilibrium Density Separation of Lymphoid Cells on Albumin and Metrizamid" at pages 102 to 117 discloses a method by which any given density band of cells in a cell population can be obtained by two simple neutral density "cuts" in two successive solutions of defined density having first established the density distribution of the cell population using continuous gradient procedures.

The techniques used by Pritchard et al. and Rolland et al. to separate the two types of SCM-responding cells from each other and from other blood components were slight modifications of the technique described by us in our 1977 European Journal of Cancer article. In this technique, the two fractions of cells are isolated in rather ill-defined bands or zones near the interface between the blood plasma and the Ficoll-Triosil density gradient. In the use of such a technique, it has proven difficult to avoid cross-contamination of the cell fractions with each other and with SCM non-responding lymphocytes. Such cross-contamination is apparent, for example, in the results shown in Table 1 of the 1982 European Journal of Cancer Clinical Oncology article by Pritchard et al., where many patients show responses to PHA in both fractions of SCM-responding cells. Since only one fraction or the other of the SCM-responding cells actually responds to PHA, depending on whether or not the cells are isolated from patients with cancer, such a dual response clearly indicates the presence of cross-contamination.

An additional disadvantage of the prior methods of lymphocyte isolation has been the use of iron powder or carbonyl-iron powder in an initial step of removing the phagocytic cells from the blood sample. The use of such materials can cause toxic effects to the lymphocytes or their hemolysis. These deleterious effects, presumably caused by impurities in some batches of the iron powder or carbonyl-iron powder, occur sporadically, but are difficult to control when they do occur.

Accordingly there is a need for a method of isolating SCM-responding lymphocytes that can be carried out rapidly by workers with limited training, that does not require special dexterity, and that can reliably separate both subpopulations of SCM-responding cells without cross-contamination or contamination of either of the sub-populations with SCM-non-responding cells. Preferably such a technique can also dispense with the use of carbonyl-iron powder or iron powder in the initial step of the procedure.

## SUMMARY

The present invention discloses improved methods for isolating either the lower buoyant density F2 subpopulation, the higher buoyant density F4 subpopulation, or both subpopulations of SCM-responding lymphocytes from a blood sample. These methods avoid cross-contamination of the SCM-responding lymphocyte subpopulations or contamination of the desired lymphocytes with non-responding lymphocytes or other blood components.

The methods can use the total population of peripheral blood lymphocytes as the starting material for the centrifugation step. Using the total population of peripheral blood lymphocytes as starting material avoids the use of carbonyl-iron powder or iron powder.

When the F2 subpopulation of lymphocytes is isolated starting with the total population of peripheral blood lymphocytes, the method comprises the steps of:

(1) layering a first solution with an osmolality of between about 0.270 Osm/kg and about 0.330 Osm/kg at 20°c and a density ($\rho_1$) determined by the equation $\rho_1 = 1.0572 + 0.063 (X - 0.290)$, where X equals the osmolality of the first solution in Osm/kg, on top of a second solution with the same osmolality as the first solution and a density ($\rho_2$) determined by the equation $\rho_2 = 1.0652 + 0.063 \, (X - 0.290)$, the volumes of the first and second solutions being sufficient to separate the F2 lymphocytes from other peripheral blood lymphocytes;

(2) layering a volume of peripheral blood lymphocytes on top of the layered first and second solutions;

(3) centrifuging the mixture of peripheral blood lymphocytes and layered first and second solutions such that the F2 lymphocyte subpopulation is concentrated in a visible band between the layered first and second solutions; and

(4) collecting the visible band to obtain the F2 lymphocyte subpopulation, the collected lymphocytes being substantially free of lymphocytes having buoyant densities greater than 1.0670 g/cm³ or less than 1.0590 g/cm³, the buoyant densities being measured at 20°C in a solution having an osmolality of about 0.315 Osm/kg.

The same method is used for isolation of the F4 lymphocyte subpopulation except that the density of the first solution is determined by the equation $\rho_1 = 1.0672 + 0.063 \, (X - 0.290)$, and the density of the second solution is determined by the equations $\rho_2 = 1.0712 + 0.063 \, (X - 0.290)$. When both the F2 and F4 subpopulations of SCM-responding lymphocytes are isolated simultaneously from the same gradient starting with the total population of peripheral blood lymphocytes, the method comprises the steps of:

(1) layering a first, second, third, and fourth solution with the same osmolality on top of each other to form a four-density solution stack:

(a) the first solution having an osmolality of between about 0.270 Osm/kg and about 0.330 Osm/kg at 20°C and a density (1) determined by the equation $\rho_1 = 1.0712 + 0.063 \, (X - 0.290)$, where X equals the osmolality of the first solution in Osm/kg;

(b) the second solution having a density ($\rho_2$) determined by the equation $2 = 1.0672 + 0.063 \, (X - 0.290)$;

(c) the third solution having a density ($\rho_3$) determined by the equation $\rho_3 = 1.0652 + 0.063 \, (X - 0.290)$; and

(d) the fourth solution having a density ($\rho_4$) determined by the equation $\rho_4 = 1.0572 + 0.063 \, (X - 0.290)$;
the first solution being positioned on the bottom of the solution stack, the volumes of the four solutions being sufficient to separate the F2 and F4 lymphocytes from each other and from other peripheral blood lymphocytes;

(2) layering a volume of peripheral blood lymphocytes on top of the solution stack;

(3) centrifuging the mixture of peripheral blood lymphocytes and the four-density solution stack such that the F2 lymphocyte subpopulation is concentrated in a visible band between the third and fourth solutions of the solution stack and the F4 lymphocyte subpopulation is concentrated in a visible band between the first and second solutions of the solution stack;

(4) collecting the visible band between the third and fourth solutions to obtain the F2 lymphocyte subpopulation, the collected lymphocytes being substantially free of lymphocytes having buoyant densities greater than 1.0670 g/cm³ or less than 1.0590 g/cm³, the buoyant densities being measured at 20°C in a solution having an osmolality of about 0.315 Osm/kg; and

(5) collecting the visible band between the first and second solutions to obtain the F4 lymphocyte subpopulation, the collected lymphocytes being substantially free of lymphocytes having buoyant densities greater than 1.0730 g/cm³ or less than 1.0690 g/cm³, the buoyant densities being measured at 20°c in a solution having an osmolality of about 0.315 Osm/kg.

In these methods, the solutions typically have an osmolality of 0.320 Osm/kg. At this osmolality, when only F2 lymphocytes are isolated, the first solution has a density of 1.0590 g/cm³ and the second solution has a density of 1.0670 g/cm³. When only F4 lymphocytes are isolated the first solution has a density of 1.0690 g/cm³ and the second solution has a density of 1.0730 g/cm³. When both F2 and F4 lymphocytes are isolated from the same gradient, the four solutions making up the solution stack have densities, respectively, of 1.0730 g/cm³, 1.0690 g/cm³, 1.0670 g/cm³, and 1.0590 g/cm³. In these methods, the two or four solutions can be solutions of polyvinylpyrrolidone-covered colloidal silica.

Any of these basic methods can further comprise the preliminary step of isolating the total population of peripheral blood lymphocytes from a heparinized blood sample by density gradient centrifugation in a medium capable of separating the peripheral blood lymphocytes from other blood components. This preliminary step can comprise the substeps of:

(1) layering heparinized blood on top of a solution of density 1.077 g/cm³ containing a non-ionic synthetic polymer of sucrose with a molecular weight of about 400,000 and sodium diatrizoate, the volume of the solution of density 1.077 g/cm³ being at least as great as the volume of heparinized blood, both solutions being equilibrated to room temperature;

(2) centrifuging the layered solutions at room temperature at sufficient effective gravitational force and for sufficient time so that the lymphocytes are banded at the interface between the solution of density 1.077 g/cm³ and the heparinized blood; and
(3) collecting the lymphocytes from the interface.

Any of these isolation methods can be combined with the SCM test, resulting in a method for testing a blood sample for the presence of a condition or a disease based upon the response of the separated lymphocytes to contact with a challenging agent. As the response to particular challenging agents of the F2 subpopulation of SCM-responding lymphocytes differs from the response of the F4 subpopulation, the challenging agent varies with the subpopulation of SCM-responding lymphocytes isolated.

When both the F2 and F4 subpopulations of SCM-responding lymphocytes are isolated, the method can comprise the steps of:

(1) heparinizing the blood sample;
(2) isolating substantially all the peripheral blood lymphocytes from the heparinized blood sample;
(3) isolating separately both the F2 and F4 subpopulations of lymphocytes from the total population of peripheral blood lymphocytes by the four-density solution stack method described above;
(4) washing the isolated subpopulations of lymphocytes, for example, twice with saline and once with Dulbecco's complete phosphate buffered saline (PBS);
(5) contacting a first aliquot of the washed F2 lymphocyte subpopulation with a substance associated with the condition or disease being tested for; and
(6) contacting the washed F4 lymphocyte subpopulation with a mitogen capable of stimulating T-cell lymphocytes.

When only the F2 subpopulation of lymphocytes is isolated, the method can comprise the steps of:

(1) heparinizing the blood sample;
(2) isolating substantially all the peripheral blood lymphocytes from the heparinized blood sample;
(3) isolating the F2 subpopulation of lymphocytes from the total population of peripheral blood lymphocytes as described above;
(4) washing the isolated F2 subpopulation of lymphocytes, for example, twice with saline and once with Dulbecco's complete phosphate buffered saline (PBS); and
(5) contacting a first aliquot of the washed F2 lymphocyte subpopulation with a substance associated with the condition or disease being tested for.

When only the F4 subpopulation of lymphocytes is isolated and used in the SCM test, the condition or disease tested for is necessarily cancer, because this lymphocyte subpopulation only shows a response in the SCM test to mitogens capable of stimulating T-cell lymphocytes and only when isolated from donors afflicted with cancer. This procedure can comprise the steps of:

(1) heparinizing the blood sample;
(2) isolating substantially all the peripheral blood lymphocytes from the heparinized blood sample;
(3) isolating the F4 subpopulation of lymphocytes from the total population of peripheral blood lymphocytes as described above;
(4) washing the isolated F4 subpopulation of lymphocytes, for example, twice with saline and once with Dulbecco's complete phosphate buffered saline (PBS); and
(5) contacting the washed F4 lymphocyte subpopulation with the mitogen.

The challenging agent used to challenge the F2 lymphocyte fraction can be a cancer associated substance. It can alternatively be selected from viral and bacterial associated antigens or produced by the onset of allograft rejection crisis. The challenging agent can also be tissue extract, in which case the contacting step comprises determining compatibility for organ transplantation between a body from which the blood sample was obtained and a body from which the tissue extract was obtained.

The mitogen capable of stimulating T-cell lymphocytes is typically reagent grade phytohaemagglutinin (PHA). When the F2 subpopulation of lymphocytes is isolated, a second aliquot of the washed F2 lymphocyte subpopulation can also be contacted with the mitogen, and the change in the SCM response of the mitogen-contacted second aliquot as a result of the contact monitored.

Similar gradient procedures can be used to isolate the F2 and/or F4 subpopulations of SCM-responding lymphocytes directly from a heparinized blood sample.

More generally, the present invention includes methods for testing a blood sample for the presence of a condition or a disease based upon the response of the lymphocytes separated from the blood sample to contact with a substance associated with the condition or disease being tested for. The lymphocyte response is indicated by changes in the SCM of the lymphocytes as a result of contact between the lymphocytes and the substance.

As stated previously, the condition or disease associated substance can be a cancer associated substance, or can be selected from viral and bacterial associated antigens. The substance can also be tissue extract or a substance produced by the onset of allograft rejection crisis.

When either the F2 fraction alone or both the F2 and F4 fractions of lymphocytes are isolated, the method can also comprise the additional steps of also contacting the isolated washed F2 fraction of cells resuspended in Dulbecco's complete PBS with the mitogen capable of stimulating T-cell lymphocytes and monitoring the response of the mitogen contacted F2 fraction to the mitogen by observing SCM changes as a result of the contact.

The present invention also includes methods for classifying and separating from a blood sample lymphocytes capable of responding to stimulation by: (a) a substance associated with a disease or condition or (b) a mitogen capable of stimulating T-cell lymphocytes. The response is exhibited by measurable changes in the SCM of the lymphocytes upon contact between the lymphocytes and the substance or the mitogen.

The F4 lymphocyte subpopulation alone can be isolated by analogous methods in a method for testing a blood sample from a donor for the presence of cancer in the donor, or in a method for classifying and separating from a blood sample lymphocytes capable of responding to stimulation by a mitogen capable of stimulating T-cell lymphocytes. Only cancer can be tested for using the F4 subpopulation of SCM-responding lymphocytes.

## DRAWINGS

These and other features, aspects, and advantages of the present invention will become better understood with reference to the following description, appended claims, and accompanying drawings where:

Fig. 1 is a plot of density versus volume measured from the meniscus of a continuous preformed density gradient solution used in the invention; and

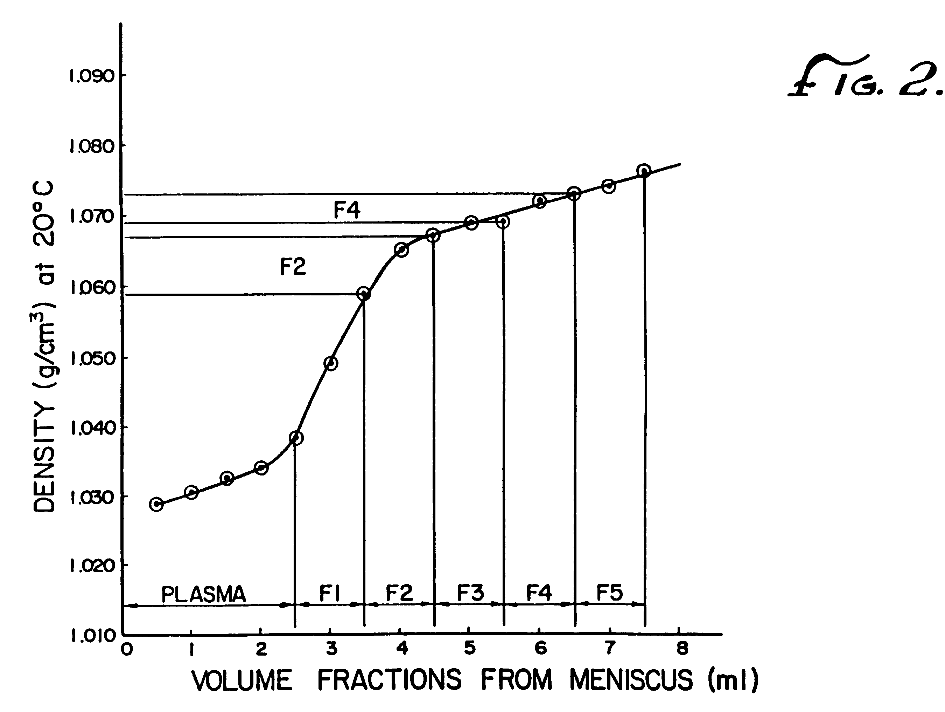

Fig. 2 is a plot of density versus the volume fractions taken from such a gradient, showing the density ranges in which the SCM-responding cells are located.

## DESCRIPTION

The procedures of the present invention can isolate either or both subsets of SCM-responding lymphocytes from a single blood sample as desired. The subset of SCM-responding lymphocytes previously studied by us, and which responds with a decrease in SCM when exposed to PHA when isolated from donors free of cancer, but responds with a decrease in SCM when exposed to cancer-associated antigens when isolated from donors with cancer, is designated the F2 lymphocyte fraction. The other subset of SCM-responding lymphocytes, which responds only to PHA and only when isolated from patients with cancer, is designated the F4 lymphocyte fraction. The F2 lymphocyte fraction has buoyant densities of from 1.0590 $g/cm^3$ to 1.0670 $g/cm^3$ as measured at 20°C at an osmolality of 0.315 Osm/kg. The F4 lymphocyte fraction has buoyant densities of from 1.0690 $g/cm^3$ to 1.0730 $g/cm^3$ under the same conditions. Lymphocytes falling outside these densities are not SCM-responding in the test procedure described herein and their presence should be avoided as diluting the effect of the SCM-responding cells. This dilution can result in a reduction in test sensitivity.

The term "solution" as used herein refers to both true solutions and to suspensions comprising partially or wholly colloidal material such as polyvinylpyrrolidone-covered colloidal silica that behave equivalently to true solutions under the centrifugation procedure of the present invention. The term "buoyant density" as used herein designates the density of that position of the multi-density gradient solution at which a particular blood component is essentially at zero gravity. At its buoyant density, the component neither exhibits buoyancy nor sinks in the gradient. Blood components, including different lymphocyte fractions, have different buoyant densities thus rendering possible their separation in the multi-density gradient solution.

As already mentioned, the buoyant densities referred to herein are taken at the standard conditions of 20°C and 0.315 Osm/kg. In certain cases it can be inconvenient to operate at standard conditions. In such cases the actual buoyant densities of the blood components that are separated at non-standard conditions are calculated back to standard conditions according to the following equations:

$$D_x = D_{0.315} + 0.063 \, (x - 0.315);$$

and

$$D_{T°} = D_{20°C} + 2.8 \times 10^{-4} \, (20°C - T°C),$$

where Dx and $D_{T^\circ}$ are buoyant densities at the osmolality of x Osm/kg and T°C, respectively.

### A. Isolation of SCM-Responding Lymphocytes

### Isolation of the SCM-Responding Lymphocytes

#### a. The Gradient

The separation method of the invention employs a multi-density gradient solution to isolate the SCM-responding lymphocytes within narrowly defined density intervals as required by the SCM test procedure outlined above. The range of densities in the gradient brackets the density intervals within which either the F2 SCM-responding lymphocytes, or the F4 SCM-responding lymphocytes, or both, occur, depending upon which cells are to be collected. If only the F2 cells are to be collected, the minimum density of the gradient is no greater than 1.050 g/cm$^3$ and the maximum density of the gradient is no less than 1.070 g/cm$^3$. If only the F4 cells are to be collected, the corresponding minimum and maximum densities are 1.060 g/cm$^3$ and 1.080 g/cm$^3$. If both the F2 and F4 cells are to be collected from a single gradient, the corresponding minimum and maximum densities are 1.050 g/cm$^3$ and 1.080 g/cm$^3$. The interval between the maximum and minimum densities optimally is not significantly greater than 0.020 g/cm$^3$ if only one fraction of SCM-responding cells is to be collected or 0.030 g/cm$^3$ if both cell fractions are to be collected. Otherwise, loss of resolution can result.

Several methods can be used to produce a multi-density gradient solution for use in the present invention. The multi-density gradient solution can be preformed by centrifugation before layering the cells on the gradient. The preformed gradient can be prepared by centrifuging a solution of an inert colloidal material such as polyvinylpyrrolidone-coated silica at sufficient effective gravitational force to form a solution having a density profile that continuously increases between the minimum and maximum densities of the gradient. For a preformed density gradient solution, the density profile is dependent on the effective gravitational force used to establish it, the temperature, and the osmolality of the solution. In general, the greater the effective gravitational force used to establish the gradient, the steeper the density profile of the gradient. We have found that the desired continuous density profile can be obtained by centrifuging the solution using a fixed 29° angle rotor at the standard conditions of temperature and osmolality at 26,000 $g_{AV}$. With a 34° angle rotor the same density profile is obtained by centrifuging at 11,400 $g_{AV}$.

Alternatively, a step gradient can be formed by layering fluids bracketing the buoyant densities of the desired F2 and/or F4 lymphocytes. If it is desired to isolate the F2 lymphocytes, a given volume of a solution of density 1.0670 g/cm$^3$ and osmolality 0.320 Osm/kg at 20°c is first placed in a centrifuge tube. On top of this solution, an equal volume of a solution of density 1.0590 g/cm$^3$ and osmolality of 0.320 Osm/kg at 20°C is layered slowly. These solutions are typically prepared from polyvinylpyrrolidone-covered silica media such as Percoll $_{(TM)}$, distributed by Pharmacia, but other density gradient media nontoxic to lymphocytes, such as Histopaque $_{(TM)}$, distributed by Sigma Diagnostics, and consisting of a nonionic synthetic polymer of sucrose in an aqueous solution of sodium diatrizoate, can also be used. If only the F4 fraction of lymphocytes is isolated, the lower solution has a density of 1.0730 g/cm$^3$ and the upper solution has a density of 1.0690 g/cm$^3$. If both the F2 and F4 fractions of lymphocytes are isolated, a four-density solution stack is prepared, with solutions of densities of 1.0730 g/cm$^3$, 1.0690 g/cm$^3$, 1.0670 g/cm$^3$, and 1.0590 g/cm$^3$ being successively layered on top of each other. The use of such a two-component or four-component step gradient is preferred when SCM-responding cells are isolated from the total lymphocyte population, as described below.

#### b. Centrifugation of Blood Samples and Collection of Lymphocyte Fractions

To separate the lymphocyte fractions, an aliquot of the blood sample depleted of phagocytic cells is layered on the multi-density gradient prepared as described above. The combination is then centrifuged at sufficient effective gravitational force to separate the blood components in the multi-density gradient solution according to their respective buoyant densities. This centrifugation is generally performed at much lower effective gravitational force than the effective gravitational force used to preform the gradient. As described in more detail in the Examples below, the effective gravitational force used to separate the lymphocytes is typically between about 550 $g_{AV}$ and about 1100 $g_{AV}$.

After centrifugation, consecutive equivolume aliquots of the multi-density gradient are withdrawn by aspiration or titration and the density of each aliquot determined. The aliquots of density corresponding to the desired F2 and/or F4 lymphocyte fractions are saved, and the desired aliquots are then washed for use in the SCM test as indicated below in the Examples.

When a step gradient with solutions of densities of 1.0670 g/cm$^3$ and 1.0590 g/cm$^3$ (for F2 lymphocytes) or 1.0730 g/cm$^3$ and 1.0690 g/cm$^3$ (for F4 lymphocytes) is used, or when a four-density solution stack is used for the isolation of both F2 and F4 lymphocytes, the desired F2 and/or F4 subpopulations isolate as visible bands, about 2 mm in height, at the interface between two solutions of differing density in the step gradient or solution stack. The F2 lymphocytes isolate between the solution of density 1.0590 g/cm$^3$ and the solution of density 1.0670 g/cm$^3$, while the F4 lymphocytes isolate between the solution of density 1.0690 g/cm$^3$ and the solution of density 1.0730 g/cm$^3$. These visible bands can be collected directly from the gradient.

### B. Isolation of SCM-Responding Lymphocytes from Total Lymphocyte Population

As an alternative to the isolation of the F2 or F4 fractions of SCM-responding lymphocytes from the entire blood sample after depletion of phagocytic cells, the fractions of SCM-responding lymphocytes can be isolated after the prior isolation of the entire population of peripheral blood lymphocytes. This not only simplifies the procedure, but also prevents possible toxic effects or hemolysis caused by impurities in some batches of the carbonyl-iron or iron powder. This variation of the procedure also eliminates slight changes in the density gradient positions of the F2 and F4 fractions of SCM-responding lymphocytes caused by differences in the hematocrit of the blood donors.

The isolation of the peripheral blood lymphocytes can be performed by a number of methods, but is preferably performed by layering a heparinized blood sample on a solution with density 1.077 g/cm$^3$ containing a nonionic synthetic polymer of sucrose with a molecular weight of about 400,000 and sodium diatrizoate, and then centrifuging the mixture. A suitable solution is Histopaque-1077 $_{(TM)}$, distributed by Sigma Diagnostics. Typically, the centrifugation is performed at 550 $g_{AV}$ for 30 minutes at room temperature. All lymphocytes banded at the interface between the density solution and the blood plasma are collected into a single centrifuge tube. The cells are then washed and resuspended in 0.9% saline for isolation of the F2 and/or F4 fraction of SCM-responding lymphocytes. Preferably, the subsequent isolation of the SCM-responding lymphocytes is carried out by the step gradient or four-density solution stack methods, as described above under "Centrifugation of Blood Samples and Collection of Lymphocyte Fractions." These methods yield the lymphocyte fractions of interest in visible bands. Collection of the SCM-responding lymphocyte fractions can be carried out manually or with an automated cell collector.

As mentioned, other methods well known in the art can be used to isolate the total lymphocyte population, such as the precipitation of erythrocytes with dextran such as isotonic T500 dextran.

### EXAMPLES

### Example 1

### Preparation of Preformed Continuous Density Gradient

A preformed continuous density gradient was prepared by mixing 56 parts by volume of a 9% (w/v) solution of polyvinylpyrrolidone-covered colloidal silica (Percoll$_{(TM)}$) marketed by Pharmacia AB) in sterile water with 44 parts per volume of a saline solution comprising 0.34 M NaCl and 0.2 g/l KH$_2$PO$_4$ in sterile preservative-free water. The density of the solution prior to centrifugation was checked with a Paar Digital Density Meter, Model DMA 55, and was found to average about 1.077 g/cm$^3$ at 20°C. The solution was sterilized by filtration through an 0.22 micron Millipore $_{(TM)}$ filter. The solution was prepared in 1000-ml quantities and could be stored for up to 2 months in a dark bottle at 4°C.

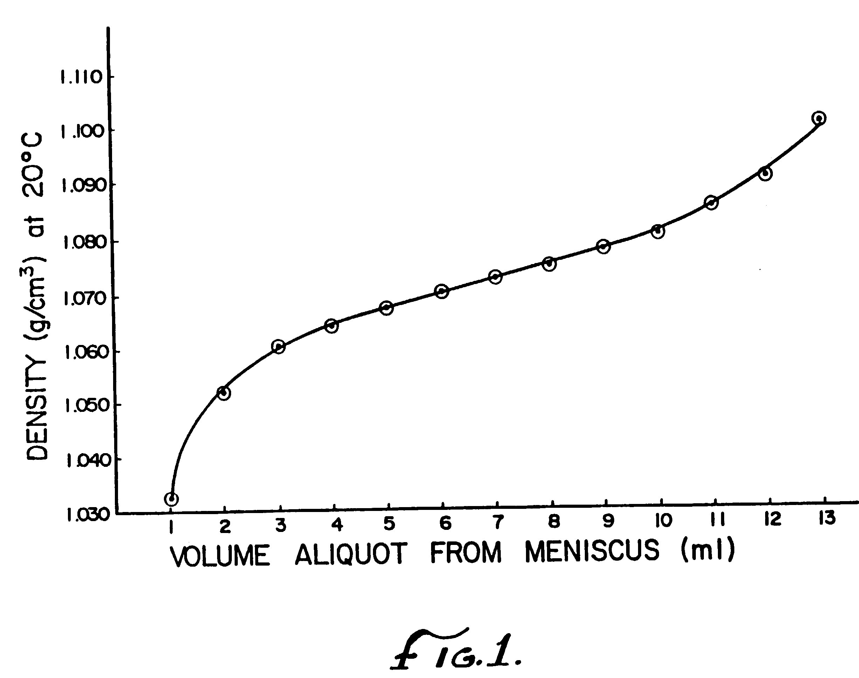

Fifteen-milliliter aliquots of this solution were transferred to glass centrifuge tubes with 16 mm O.D. and brought to a temperature of 20°C. The aliquots were centrifuged for 30 minutes at 26,000 $g_{AV}$ using a fixed 29° angle rotor to preform the gradient. The density profile of the solution after centrifugation was determined by measuring the density of each consecutive 1 ml fraction at 20°C using the Paar Digital Density Meter. A plot of the density of each of the consecutive fractions is shown in Fig. 1. As shown, there was a continuous increase in the density of the fractions, from the second volume fraction at a density of about 1.053 g/cm$^3$ to the twelfth volume fraction at a density of about 1.090 g/cm$^3$.

### Comparative Example 2

### Separation of SCM-Responding Lymphocytes Using the Gradient of Example 1

Twenty-milliliter samples of peripheral blood were obtained from 30 healthy donors and 20 donors who had been positively diagnosed as having a malignancy. For the purposes of the example, the specific type of cancer is not critical.

The blood samples were collected in LH/10 lithium heparin-containing blood collection tubes distributed by Searle or in heparinized Vacutainer$_{(TM)}$ tubes. Heparinized blood samples can be stored at room temperature (18°C to 20°C) for up to 12 hours. Ten milliliters of each blood sample was transferred to a separate 20 ml glass vial containing 0.1 g carbonyl-iron powder (type SF distributed by GAF, Ltd., Great Britain). Equivalent results have been achieved using iron powder distributed by Koch-Light Laboratories, Ltd. and identified as 8365 x 99.5%. The vials were rotated at 30 revolutions per minute for 30 minutes at 37°C and then placed on a magnet for 15-30 minutes to effect separation of the phagocytic cells along with the iron powder from the blood sample.

A four-to-five milliliter aliquot of each of the blood samples depleted of phagocytes was transferred to a centrifuge tube containing the continuous density gradient solution prepared in Example 1 and from which the top 4 ml of the density gradient solution had been removed. Both the density gradient solution and the blood aliquot had been equilibrated to a temperature of 20°C before the transfer. The transfer was accomplished by layering the blood on top of the density gradient solution. The tubes were placed in a centrifuge that was thermostatically controlled to maintain a temperature of about 20°C and gradually accelerated over 1 minute to 550 $g_{AV}$ and held at that speed for 30 minutes. No brake was applied during deceleration. Consecutive 0.5 ml aliquots were removed from the tubes and the density of each aliquot was determined at 20°C with the Paar Digital Density Meter.

Examination of consecutive 0.5 ml aliquots removed from each of the centrifuge tubes after centrifugation showed that the first five aliquots removed, representing the top 2.5 ml of the gradient, contained essentially cell-free plasma. The next two aliquots, representing that portion of the gradient from 2.5 ml to 3.5 ml, contained approximately 98% lymphocytes. These two aliquots were combined and designated fraction 1 (F1). The blood components isolated in the next two aliquots, spanning that portion of the gradient from 3.5 ml to 4.5 ml, comprised nearly 100% lymphocytes. These two aliquots were combined as fraction 2 (F2). The third fraction (F3) was a combination of the next two aliquots of the gradient, spanning that portion of the gradient from 4.5 ml to 5.5 ml. This fraction contained about 99% lymphocytes. The fourth fraction (F4) was a combination of the next two aliquots of the gradient from 5.5 ml to 6.5 ml, and contained about 80% lymphocytes. The last fraction collected, fraction 5 (F5), was a combination of the next two aliquots of the gradient from 6.5 ml to 7.5 ml, and contained about 75% lymphocytes. The remaining aliquots of the gradient contained mainly erythrocytes and were discarded. The blood components other than lymphocytes in the fractions F1-F5 comprised mostly erythrocytes, although some granulocytes or platelets were also observed.

The density range of each of the fractions was taken as the minimum density of the first aliquot forming the fraction to the maximum density of the second aliquot forming the fraction. Table 1 is a summary of the density ranges and the percentage of lymphocytes in the blood components in fractions 1-5 as collected from the density gradient.

## TABLE 1

## DENSITY RANGES OF FRACTIONS TAKEN FROM GRADIENT OF EXAMPLE

## 2 AND PERCENTAGES OF LYMPHOCYTES IN FRACTIONS

| Fraction No. | Aliquot, ml[a] | Density, g/cm$^3$ | % Lymphocytes |
|---|---|---|---|
| 1 | 2.5-3.5 | 1.0385-1.0590 | 98.6 |
| 2 | 3.5-4.5 | 1.0590-1.0670 | 99.7 |
| 3 | 4.5-5.5 | 1.0670-1.0690 | 99.4 |
| 4 | 5.5-6.5 | 1.0690-1.0730 | 80.0 |
| 5 | 6.5-7.5 | 1.0730-1.0760 | 75.0 |

Densities were measured at 20°C and at an osmolality of 0.315-0.320 Osm/kg.

[a] Measurement of aliquots taken was from meniscus of gradient.

Example 3

Isolation of Total Population of Peripheral Blood Lymphocytes

For this separation, 20 ml of heparinized blood (treated with 300 I.V. of heparin/10 ml of blood) was layered in 5-ml aliquots on 5-ml aliquots of Histopaque 1077$_{(TM)}$ (distributed by Sigma Diagnostics) in 15-ml (16 mm O.D.) centrifuge tubes. Both the blood and the Histopaque solution were equilibrated to room temperature before layering. The layered solutions were then centrifuged at 550 $g_{AV}$ for 30 minutes at room temperature. All lymphocytes isolated at the interface between the density solution and the blood plasma were collected into a single centrifuge tube. The collected cells were washed once with 0.9% saline, pelleted by centrifugation at 550 $g_{AV}$ for 5 to 10 minutes, and resuspended in 4 ml of 0.9% saline for the second isolation step, the isolation of the SCM-responding fraction of lymphocytes.

Example 4

Isolation of F2 Fraction of SCM-Responding Lymphocytes from Total Population of Peripheral Blood Lymphocytes

Two density solutions, prepared from Percoll$_{(TM)}$ (Pharmacia AB), were used for isolation of the F2 fraction of SCM-responding lymphocytes from the total population of peripheral blood lymphocytes (Example 3). The step gradient used was prepared as follows: First, four milliliters of a density solution with a density of 1.0670 g/cm$^3$ at 20°C and an osmolality of 0.320 Osm/kg (Solution A) was dispensed into a 15-ml glass centrifuge tube with an O.D. of 16 mm. On top of this solution, four milliliters of a density solution with a density of 1.0590 g/cm$^3$ and osmolality of 0.320 Osm/kg (Solution B) was layered slowly to form a bilayer of density solutions. On top of this bilayer, four milliliters of the suspension of the total population of peripheral blood lymphocytes from Example 3 was layered. Both the bilayer and the suspension of lymphocytes were equilibrated to 20° ± 1°C.

The F2 sub-population of SCM-responding lymphocytes isolated as a visible band, about 2 mm in height, between the density solutions A and B, and the band was collected directly. After collection, the lymphocytes were washed twice with preservative-free 0.9% saline for injections, then once or twice with Dulbecco's complete phosphate buffered saline (PBS), and then resuspended in 1 to 2 ml of PBS.

Example 5

Isolation of Both F2 and F4 Fractions of SCM-Responding Lymphocytes from Total Population of Peripheral Blood Lymphocytes

The procedure of Example 4 was used, except that the lymphocyte suspension of Example 3 was layered on a four-density solution stack prepared by layering on top of each other 3 ml aliquot of Percoll solutions with the following densities at 20°C and osmolalities of 0.320 Osm/kg: 1.0730 g/cm$^3$ (Solution D); 1.0690 g/cm$^3$ (Solution C); 1.0670 g/cm$^3$ (Solution B); and 1.0590 g/cm$^3$ (Solution A), with solution D on the bottom of the stack. When this four density solution stack was used, the F2 sub-population of SCM-responding lymphocytes was isolated as a visible band at the interface between Solution A and B, while the F4 sub-population was isolated as a visible band at the interface between solutions C and D.

Example 6

Determination of SCM Response to Cancer-Associated Antigen

Each fraction obtained in Comparative Example 2 was checked for the SCM response of the lymphocytes in the fraction in accordance with the SCM cancer screening procedure as described in the article by L. Cercek and B. Cercek, "Application of the Phenomenon of Changes in the Structuredness of Cytoplasmic Matrix (SCM) in the Diagnosis of Malignant Disorders: A Review," Europ. J. Cancer 13, 903-915 (1977). The procedure used is described in International Publication no WO87/05393 at page 15 line 27 to page 18 line 35.

For each of the five lymphocyte fractions, F1 to F5, whose isolation was described in Comparative Example 2, the mean polarization or P values for samples stimulated with PHA and cancer-associated antigen were determined as a percentage of the mean P value of stimulated control samples from each of the fractions as isolated from 30 healthy donors and from 20 donors who had been positively diagnosed as having a malignancy. These results are summarized in Table 2 below.

Table 2 shows that the lymphocytes present in fractions 1, 3, and 5 of the continuous density gradient were SCM non-responding. For the cells of fractions 1 and 3, there was essentially no response to simulation by either PHA or the cancer-associated antigen. For the cells of fraction 5, the lymphocytes from healthy donors exhibited no response to the mitogen, indicating that the cells were also SCM non-responding. This was confirmed by the failure of these cells to exhibit a fluorescence polarization peak at 510 nm, in contrast to the cells of fractions 2 and 4. The cells of fraction 2 (F2 cells) exhibited classic SCM responses to stimulation by PHA and cancer-associated antigen. For example, F2 lymphocytes from healthy donors had a marked percentage decrease in polarization value when stimulated by PHA as compared to unstimulated control cells from the same fraction. This indicated an SCM response by the lymphocytes to PHA. These same lymphocytes had essentially no SCM response to the cancer-associated antigen. Similarly, F2 lymphocytes from donors afflicted with cancer had no SCM response to PHA but had a definite SCM response to the cancer-associated antigen. This shows that F2 lymphocytes, which have a buoyant density of 1.0590 $g/cm^3$ to 1.0670 $g/cm^3$ at 20°C and an osmolality of 0.315 Osm/kg, are suitable for use in the SCM test for the detection of cancer.

The F4 lymphocytes, which have a buoyant density of 1.0690 $g/cm^3$-1.0730 $g/cm^3$ at 20°C and an osmolality of 0.315 Osm/kg, also demonstrated an SCM response to PHA that would indicate usefulness in the SCM cancer test. However, the response was markedly different than for F2 lymphocytes. In contrast to F2 lymphocytes, F4 lymphocytes from donors afflicted with cancer had a significant SCM response to PHA, but the equivalent fraction of lymphocytes from donors free of cancer had no response to PHA. In addition, F4 cells exhibited no response to cancer-associated antigen regardless of the donor's condition. It should be noted that the response of the F4 lymphocytes of PHA was the opposite to the response of F2 cells to PHA. Although the reason for this is not understood, F4 lymphocytes can be used in the SCM test to screen blood samples for the presence of cancer. A positive SCM response by a donor's F4 lymphocytes provides a positive indication of the presence of a malignancy, calling for further testing and diagnosis.

## TABLE 2

### SCM RESPONSE OF LYMPHOCYTE FRACTIONS OF EXAMPLE 2 TO PHA AND CANCER ASSOCIATED ANTIGEN (CAA) AS PERCENTAGE OF CONTROL

### (EXAMPLE 6)

| Fraction No. | PHA[a] | CAA[a] | PHA[b] | CAA[b] |
|---|---|---|---|---|
| 1 | 99.9 ± 1.5 | 100 ± 2.0 | 100 ± 2.0 | 99.8 ± 1.5 |
| 2 | 71 ± 6.0 | 99 ± 3.0 | 98 ± 2.0 | 74 ± 6.0 |
| 3 | 101 ± 2.0 | 100 ± 2.0 | 100 ± 2.0 | 100 ± 2.0 |
| 4 | 99 ± 2.5 | 100 ± 2.0 | 74 ± 8.0 | 99 ± 3.0 |
| 5 | 100 ± 1.5 | --------- | 99 ± 2.0 | ---------- |

[a] Each value is the mean of 30 samples from healthy donors.

[b] Each value is the mean of 20 samples from donors diagnosed as having a malignant disorder.

These results clearly show that F4 lymphocytes must be separated from F2 lymphocytes prior to stimulation by a challenging agent, particularly if a mitogen such as PHA is used as the challenging agent. The presence of both F2 and F4 cells in the same SCM assay leads to erroneous results, indicating the absence of malignancy when it is in fact present or vice versa. The improved separation technique of the present invention clearly separates both classes of cells from each other and from SCM non-responding cells. This prevents the possibility of occurrence of such erroneous results.

Example 7

Use of SCM Response to Determine Allograft Rejection and Tissue Compatibility

F2 lymphocytes separated in accordance with the procedure of Comparative Example 2 from the peripheral blood of an organ transplant recipient are used to indicate the onset of allograft rejection crisis. Utilizing the SCM-response technique of Example 6, the separated F2 lymphocytes are incubated with a tissue extract in PBS obtained from tissue of the organ donor. Organ rejection is indicated when the F2 lymphocytes from the organ recipient show a decrease in intracellular fluorescein fluorescence polarization after contact with the tissue extract from the organ donor. Compatibility is indicated by no decrease in the polarization value of the recipient's F2 lymphocytes when the donor's tissue extract is used as a challenging agent in the SCM test.

As an alternative to the use of tissue extract as a challenging agent, the F2 lymphocytes of the recipient are incubated with a cell-free extract or essentially cell-free extract (containing about 5% cells) in PBS of F2 lymphocytes from the blood of the organ donor, and the resulting SCM response is measured as in Example 6.

The technique of this Example is also utilized prior to the organ transplant to determine compatibility between the organ donor and the potential organ recipient. The procedure of this Example is carried in the relatively short time of 3 to 4 hours and is a timesaving alternative to the conventional mixed lymphocyte reaction procedure. The latter procedure is conventionally used as a measure of compatibility between a donor and a recipient and takes between 48 and 72 hours to obtain results.

Example 8

SCM Response to Determine Allergic Reaction

F2 lymphocytes separated according to Comparative Example 2 from the peripheral blood of a donor suffering from an allergic reaction are used according to the method of Example 6 to determine the reaction of the donor to particular allergens. The allergens can be pollen extract, dust extract, animal hair extract, grass extracts, food extracts, bee toxins, and the like as are presently used in conventional skin patch tests. An allergic response to a particular antigen is indicated by the method of Example 6 by a decrease in the intracellular fluorescein fluorescence polarization value of the donor's F2 lymphocytes. Such a decrease indicates an SCM response by the F2 lymphocytes to a particular allergen. The use of this method can avoid the necessity for painful and inconvenient skin patch tests.

Example 9

SCM Response to Determine Viral or Bacterial Infection

F2 lymphocytes are isolated according to the method of Comparative Example 2 from the peripheral blood of a donor afflicted with an unknown viral or bacterial infection. The F2 cells are incubated according to the method of Example 6 with extracts or toxins of viruses or bacteria which are suspected to be the cause of the infection. The F2 lymphocytes respond in the SCM test by a decrease in intracellular fluorescein fluorescence polarization only when incubated with the specific toxin or virus that is present in the body of the donor and not when incubated with any other agents.

The SCM-responding lymphocyte isolation method of the present invention meets the needs previously set forth: rapid isolation of SCM-responding lymphocytes, suitability for use by workers with limited training, lack of a requirement for complex manipulations or special dexterity, and reliable separation of the two subpopulations of SCM-responding lymphocytes without cross-contamination or contamination of either of the fractions with SCM non-responding cells.

Additionally, one embodiment of the method eliminates the need for using iron powder or carbonyl-iron powder in a preliminary step of the isolation procedure, simplifying the procedure and avoiding the use of a reagent with possible toxic or hemolytic effects on the lymphocytes.

The method of the present invention allows the isolation of either the F2 subpopulation or the F4 subpopulation of SCM-responding lymphocytes, or both, from the same gradient. It isolates the SCM-responding lymphocytes as well-defined bands, cleanly separated from other blood components, such as non-SCM-responding lymphocytes. The use of this method can improve the reliability of the SCM test and its utility in clinical applications.

Although the present invention has been described in considerable detail with regard to certain preferred versions thereof, other versions are possible.

**Claims**

1. A method for isolating the F2 subpopulation of SCM-responding lymphocytes from the total population of peripheral blood lymphocytes isolated from blood comprising the steps of:

(a) layering a first solution having an osmolality of between about 0.270 Osm/kg and about 0.330 Osm/kg at 20°C and a density ($\rho_1$) determined by the equation $\rho_1 = 1.0572 + 0.063 (X - 0.290)$, where X equals the osmolality of the first solution in Osm/kg, on top of a second solution with the same osmolality as the first solution and a density ($\rho_2$) determined by the equation $\rho_2 = 1.0652 + 0.063\,(X - 0.290)$, the volumes of the first and second solutions being sufficient to separate the F2 lymphocytes from other peripheral blood lymphocytes;

(b) layering a volume of peripheral blood lymphocytes on top of the layered first and second solutions;

(c) centrifuging the peripheral blood lymphocytes layered on top of the layered first and second solutions such that the F2 lymphocyte subpopulation is concentrated in a visible band between the layered first and second solutions; and

(d) collecting the visible band to obtain the F2 lymphocyte subpopulation, the collected lymphocytes being substantially free of lymphocytes having buoyant densities greater than about 1.0670 g/cm$^3$ or less than about 1.0590 g/cm$^3$, the buoyant densities being measured at 20°C in a solution having an osmolality of about 0.315 Osm/kg.

2. A method for isolating both the F2 and F4 subpopulations of SCM-responding lymphocytes from the total population of peripheral blood lymphocytes isolated from blood comprising the steps of:

(a) layering a first, second, third, and fourth solution having the same osmolality on top of each other to form a four-density solution stack:

(i) the first solution having an osmolality of between about 0.270 Osm/kg and about 0.330 Osm/kg at 20°C and a density ($\rho_1$) determined by the equation $\rho_1 = 1.0712 + 0.063\,(X - 0.290)$, where X equals the osmolality of the first solution in Osm/kg,;

(ii) the second solution having a density ($\rho_2$) determined by the equation $\rho_2 = 1.0672 + 0.063\,(X - 0.290)$;

(iii) the third solution having a density ($\rho_3$) determined by the equation $\rho_3 = 1.0652 + 0.063\,(X - 0.290)$;

(iv) the fourth solution having a density ($\rho_4$) determined by the equation $\rho_4 = 1.0572 + 0.063\,(X - 0.290)$;

the first solution being positioned on the bottom of the solution stack, the volumes of the four solutions being sufficient to separate the F2 and F4 lymphocytes from each other and from other peripheral blood lymphocytes;

(b) layering a volume of peripheral blood lymphocytes on top of the solution stack;

(c) centrifuging the peripheral blood lymphocytes layered on top of the four-density solution stack such that the F2 lymphocyte subpopulation is concentrated in a visible band between the third and fourth solutions of the solution stack and the F4 lymphocyte subpopulation is concentrated in a visible band between the first and second solutions of the solution stack;

(d) collecting the visible band between the third and fourth solutions to obtain the F2 lymphocyte subpopulation, the collected lymphocytes being substantially free of lymphocytes having buoyant densities greater than about 1.0670 g/cm$^3$ or less than about 1.0590 g/cm$^3$, the buoyant densities being measured at 20°C in a solution having an osmolality of about 0.315 Osm/kg; and

(e) collecting the visible band between the first and second solutions to obtain the F4 lymphocyte subpopulation, the collected lymphocytes being substantially free of lymphocytes having buoyant densities greater than about 1.0730 g/cm$^3$ or less than about 1.0690 g/cm$^3$, the buoyant densities being measured at 20°C in a solution having an osmolality of about 0.315 Osm/kg.

3. A method for isolating the F4 subpopulation of SCM-responding lymphocytes from the total population of peripheral blood lymphocytes isolated from blood comprising the steps of:

(a) layering a first solution having an osmolality of between about 0.270 Osm/kg and about 0.330 Osm/kg at 20°C and a density ($\rho_1$) determined by the equation $\rho_1 = 1.0672 + 0.063\,(X - 0.290)$, where X equals the osmolality of the first solution in Osm/kg, on top of a second solution having the same osmolality as the first solution and a density ($\rho_2$) determined by the equation $\rho_2 = 1.0712 + 0.063\,(X - 0.290)$, the volumes of the first and second solutions being sufficient to separate the F4 lymphocytes from other peripheral blood lymphocytes;

(b) layering a volume of peripheral blood lymphocytes on top of the layered first and second solutions;

(c) centrifuging the peripheral blood lymphocytes layered on top of the layered first and second solutions such that the F4 lymphocyte subpopulation is concentrated in a visible band between the layered first and second solutions; and

(d) collecting the visible band to obtain the F4 lymphocyte subpopulation, the collected lymphocytes being substantially free of lymphocytes having buoyant densities greater than about 1.0730 g/cm$^3$ or less than about 1.0690 g/cm$^3$, the buoyant densities being measured at 20°C in a solution having an osmolality of about 0.315 Osm/kg.

4. The method of claim 1 wherein the osmolality of the first and second solutions is 0.320 Osm/kg, the first solution has a density of 1.0590 g/cm$^3$, and the second solution has a density of 1.0670 g/cm$^3$.

5. The method of claim 2 wherein the osmolality of the solutions forming the four-density solution stack is 0.320 Osm/kg, the first solution has a density of 1.0730 g/cm$^3$, the second solution has a density of 1.0690 g/cm$^3$, the third solution has a density of 1.0670 g/cm$^3$, and the fourth solution has a density of 1.0590 g/cm$^3$.

6. The method of claim 3 wherein the osmolality of the first and second solutions is 0.320 Osm/kg, the first solution has a density of 1.0690 g/cm$^3$, and the second solution has a density of 1.0730 g/cm$^3$.

7. The method of claim 1 or 3 wherein the first and second solutions are solutions of polyvinylpyrrolidone-covered colloidal silica.

8. The method of claim 2 wherein the four solutions are solutions of polyvinylpyrrolidone-covered colloidal silica.

9. The method of claim 1, 2, or 3 further comprising the step of isolating the total population of peripheral blood lymphocytes from a heparinized blood sample by density gradient centrifugation in a medium capable of separating the peripheral blood lymphocytes from other blood components.

10. The method of claim 9 wherein the step of isolating the total population of peripheral blood lymphocytes comprises the steps of:

(i) layering heparinized blood on top of a solution of density 1.077 g/cm$^3$ containing a nonionic synthetic polymer of sucrose with a molecular weight of about 400,000 and sodium diatrizoate, the volume of the solution of density 1.077 g/cm$^3$ being at least as great as the volume of heparinized blood, both solutions being equilibrated to room temperature;
(ii) centrifuging the layered solutions at room temperature at sufficient effective gravitational force and for sufficient time so that the lymphocytes are banded at the interface between the solution of density 1.077 g/cm$^3$ and the heparinized blood; and
(iii) collecting the lymphocytes from the interface.

11. A method for testing a blood sample for the presence of a condition or a disease comprising the steps of:

(a) heparinizing the blood sample;
(b) isolating substantially all the peripheral blood lymphocytes from the heparinized blood sample;
(c) isolating separately both the F2 and F4 subpopulations of lymphocytes from the total population of peripheral blood lymphocytes by the steps of:

(i) layering a first, second, third, and fourth solution with the same osmolality on top of each other to form a four-density solution stack:

[a] the first solution having an osmolality of between about 0.270 Osm/kg and about 0.330 Osm/kg at 20°C and a density ($\rho_1$) determined by the equation $\rho_1 = 1.0712 + 0.063\,(X - 0.290)$, where X equals the osmolality of the first solution in Osm/kg;
[b] the second solution having a density ($\rho_2$) determined by the equation $\rho_2 = 1.0672 + 0.063\,(X - 0.290)$;
[c] the third solution having a density ($\rho_3$) determined by the equation $\rho_3 = 1.0652 + 0.063\,(X - 0.290)$; and
[d] the fourth solution having a density ($\rho_4$) determined by the equation $\rho_4 = 1.0572 + 0.063\,(X - 0.290)$; the first solution being positioned on the bottom of the solution stack, the volumes of the four solutions being sufficient to separate the F2 and F4 lymphocytes from each other and from other peripheral blood lymphocytes;

(ii) layering a volume of peripheral blood lymphocytes on top of the solution stack;
(iii) centrifuging the peripheral blood lymphocytes layered on top of the four-density solution stack such that the F2 lymphocyte subpopulation is concentrated in a visible band between the third and fourth solutions of the solution stack and the F4 lymphocyte subpopulation is concentrated in a visible band between the first and second solutions of the solution stack;
(iv) collecting the visible band between the third and fourth solutions to obtain the F2 lymphocyte subpopulation, the collected lymphocytes being substantially free of lymphocytes having buoyant densities greater than about 1.0670 $g/cm^3$ or less than about 1.0590 $g/cm^3$, the buoyant densities being measured at 20°C in a solution having an osmolality of about 0.315 Osm/kg; and

(v) collecting the visible band between the first and second solutions to obtain the F4 lymphocyte subpopulation, the collected lymphocytes being substantially free of lymphocytes having buoyant densities greater than about 1.0730 $g/cm^3$ or less than about 1.0690 $g/cm^3$, the buoyant densities being measured at 20°C in a solution having an osmolality of about 0.315 Osm/kg;

(d) washing the isolated subpopulations of lymphocytes;

(e) adding a fluorogenic agent precursor to each of the washed, isolated lymphocyte subpopulations;

(f) contacting an aliquot of the washed F2 lymphocyte subpopulation to which the fluorogenic agent precursor has been added with a substance associated with the condition of disease being tested for.

(g) contacting the washed F4 lymphocyte subpopulation to which the fluorogenic agent precursor has been added with a mitogen capable of stimulating T-cell lymphocytes;

(h) monitoring the response of the aliquot of the F2 lymphocyte subpopulation contacted with the substance by observing the changes in the structuredness of the cytoplasmic matrix of the lymphocytes of the aliquot as a result of the contact between the lymphocytes and the substance;

(i) monitoring the response of the F4 lymphocyte subpopulation contacted with the mitogen by observing the changes in the structuredness of the cytoplasmic matrix of the lymphocytes as a result of the contact between the lymphocytes and the mitogen; and

(j) relating the monitored response of the aliquot of the F2 lymphocyte subpopulation and the F4 lymphocyte subpopulation to the presence or absence of the disease or bodily condition.

12. A method for testing a blood sample for the presence of a condition or a disease comprising the steps of:

(a) heparinizing the blood sample;

(b) isolating substantially all the peripheral blood lymphocytes from the heparinized blood sample;

(c) isolating the F2 subpopulation of lymphocytes from the total population of peripheral blood lymphocytes by the steps of:

(i) layering a first solution having an osmolality of between about 0.270 Osm/kg and about 0.330 Osm/kg at 20°C and a density ($\rho_1$) determined by the equation $\rho_1 = 1.0572 + 0.063 (X - 0.290)$, where $X$ equals the osmolality of the first solution in Osm/kg, on top of a second solution with the same osmolality as the first solution and a density ($\rho_2$) determined by the equation $\rho_2 = 1.0652 + 0.063 (X - 0.290)$, the volumes of the first and second solutions being sufficient to separate the F2 lymphocytes from other peripheral blood lymphocytes;

(ii) layering a volume of peripheral blood lymphocytes on top of the layered first and second solutions;

(iii) centrifuging the peripheral blood lymphocytes layered on top of the layered first and second solutions such that the F2 lymphocyte subpopulation is concentrated in a visible band between the layered first and second solutions; and

(iv) collecting the visible band to obtain the F2 lymphocyte subpopulation, the collected lymphocytes being substantially free of lymphocytes having buoyant densities greater than about 1.0670 $g/cm^3$ or less than about 1.0590 $g/cm^3$, the buoyant densities being measured at 20°C in a solution having an osmolality of about 0.315 Osm/kg;

(d) washing the isolated F2 subpopulation of lymphocytes;

(e) adding a fluorogenic agent precursor to the washed, isolated F2 lymphocyte subpopulation;

(f) contacting an aliquot of the washed F2 lymphocyte subpopulation to which the fluorogenic agent precursor has been added with a substance associated with the condition or disease being tested for.

(g) monitoring the response of the aliquot of the F2 lymphocyte subpopulation contacted with the substance by observing the changes in the structuredness of the cytoplasmic matrix of the lymphocytes of the aliquot as a result of the contact between the lymphocytes and the substance; an

(h) relating the monitored response of the aliquot of the F2 lymphocyte subpopulation to the presence or absence of the disease or bodily condition.

13. A method for testing a blood sample from a donor for the presence of cancer in the donor comprising the steps of:

(a) heparinizing the blood sample;

(b) isolating substantially all the peripheral blood lymphocytes from the heparinized blood sample;
(c) isolating the F4 subpopulation of lymphocytes from the total population of peripheral blood lymphocytes by the steps of:

(i) layering a first solution having an osmolality of between about 0.270 Osm/kg and about 0.330 Osm/kg at 20°C and a density ($\rho_1$) determined by the equation $\rho_1 = 1.0672 + 0.063 (X - 0.290)$, where X equals the osmolality of the first solution in Osm/kg, on top of a second solution having the same osmolality as the first solution and a density ($\rho_2$) determined by the equation $\rho_2 = 1.0712 + 0.063 (X - 0.290)$, the volumes of the first and second solutions being sufficient to separate the F4 lymphocytes from other peripheral blood lymphocytes;
(ii) layering a volume of peripheral blood lymphocytes on top of the layered first and second solutions;
(iii) centrifuging the peripheral blood lymphocytes layered on top of the layered first and second solutions such that the F4 lymphocyte subpopulation is concentrated in a visible band between the layered first and second solutions; and
(iv) collecting the visible band to obtain the F4 lymphocyte subpopulation, the collected lymphocytes being substantially free of lymphocytes having buoyant densities greater than about 1.0730 $g/cm^3$ or less than about 1.0690 $g/cm^3$, the buoyant densities being measured at 20°C in a solution having an osmolality of about 0.315 Osm/kg;

(d) washing the isolated F4 subpopulation of lymphocytes;
(e) adding a fluorogenic agent precursor to the washed, isolated F4 lymphocyte subpopulation;
(f) contacting the washed F4 lymphocyte subpopulation to which the fluorogenic agent precursor has been added with the mitogen;
(g) monitoring the response of the F4 lymphocyte subpopulation contacted with the mitogen by observing the changes in the structuredness of the cytoplasmic matrix of the lymphocytes of the aliquot as a result of the contact between the lymphocytes and the mitogen; and
(h) relating the monitored response of the F4 lymphocyte subpopulation to the presence or absence of cancer.

14. The method of claim 11 or claim 12 wherein the condition or disease associated substance is a cancer associated substance.

15. The method of claim 11 wherein the condition or disease associated substance is selected from the group consisting of viral associated antigens and bacterial associated antigens.

16. The method of claim 11 wherein the condition or disease associated substance is capable of causing a decrease in the structuredness of the cytoplasmic matrix of lymphocytes incompatible with the donor of the lymphocytes producing the substance and is present as an indicator of allograft rejection crisis.

17. The method of claim 11 wherein the substance is tissue extract from a transplant donor and the step of relating the monitored response comprises determining compatibility for organ transplantation between a transplant recipient, from which the blood sample was obtained, and the transplant donor, from whom the tissue extract was obtained.

18. The method of claim 11 comprising the additional steps of:

(a) also contacting a second aliquot of the washed F2 lymphocyte subpopulation to which the fluorogenic agent precursor has been added with the mitogen capable of stimulating T-cell lymphocytes;
(b) monitoring the response of the mitogen-contacted second aliquot of the F2 lymphocyte subpopulation by observing the changes in the structuredness of the cytoplasmic matrix of the lymphocytes of the aliquot as a result of the contact between the lymphocytes and the mitogen; and
(c) relating the monitored response of the second aliquot of the F2 lymphocyte subpopulation to the presence or absence of the disease or bodily condition to confirm the result obtained with the first aliquot of the F2 lymphocyte subpopulation.

19. The method of claim 12 comprising the additional steps of:

(a) also contacting a second aliquot of the washed F2 lymphocyte subpopulation to which the fluorogenic agent precursor has been added with a mitogen capable of stimulating T-cell lymphocytes;
(b) monitoring the response of the mitogen-contacted second aliquot of the F2 lymphocyte subpopulation to the mitogen by observing the changes in the structuredness of the cytoplasmic matrix of the lymphocytes of the second aliquot as a result of the contact between the lymphocytes and the mitogen; and

(c) relating the monitored response of the second aliquot of the F2 lymphocyte subpopulation to the presence or absence of the disease or bodily condition to confirm the result obtained with the first aliquot of the F2 lymphocyte subpopulation and with the F4 lymphocyte subpopulation.

20. The method of claim 11, 13, 18, or 19 wherein the mitogen is reagent grade phytohaemagglutinin.

21. The method of claim 11 wherein the osmolality of the solutions forming the four-density solution stack is 0.320 Osm/kg, the first solution has a density of 1.0730 $g/cm^3$, the second solution has a density of 1.0690 $g/cm^3$, the third solution has a density of 1.0670 $g/cm^3$, and the fourth solution has a density of 1.0590 $g/cm^3$.

22. The method of claim 12 wherein the osmolality of the first and second solutions is 0.320 Osm/kg, the first solution has a density of 1.0590 $g/cm^3$, and the second solution has a density of 1.0670 $g/cm^3$.

23. The method of claim 13 wherein the osmolality of the first and second solutions is 0.320 Osm/kg, the first solution has a density of 1.0690 $g/cm^3$, and the second solution has a density of 1.0730 $g/cm^3$.

24. The method of claim 11, 12, or 13 wherein the step of isolating substantially all the peripheral blood lymphocytes from the heparinized blood sample comprises the steps of:

(i) layering heparinized blood on top of a solution of density 1.077 $g/cm^3$ containing a nonionic synthetic polymer of sucrose with a molecular weight of about 400,000 and sodium diatrizoate, the volume of the solution of density 1.077 $g/cm^3$ being at least as great as the volume of heparinized blood, both solutions being equilibrated to room temperature;
(ii) centrifuging the layered solutions at room temperature at sufficient effective gravitational force and for sufficient time so that the lymphocytes are banded at the interface between the solution of density 1.077 $g/cm^3$ and the heparinized blood; and
(iii) collecting the lymphocytes from the interface.

25. The method of claim 11 wherein the four solutions are solutions of polyvinylpyrrolidone-covered colloidal silica.

26. The method of claim 12 or 13 wherein the first and second solutions are solutions of polyvinylpyrrolidone-covered colloidal silica.

**Patentansprüche**

1. Verfahren zur Isolierung der F2-Subpopulation von SCM-responsiven Lymphozyten aus der aus Blut isolierten Gesamtpopulation an peripheren Blutlymphozyten, folgende Schritte umfassend;

(a) Aufschichten einer ersten Lösung mit einer Osmolalität zwischen etwa 0,270 Osm/kg und etwa 0,330 Osm/kg bei 20°C und einer Dichte ($\rho_1$), welche durch die Gleichung $\rho_1 = 1,0572 + 0,063 \, (X - 0,290)$, worin X der Osmolalität der ersten Lösung in Osm/kg entspricht, bestimmt ist, auf eine zweite Lösung mit der gleichen Osmolalität wie die erste Lösung und einer Dichte ($\rho_2$), die durch die Gleichung $\rho_2 = 1,0652 + 0.063 \, (X - 0,290)$ bestimmt ist, wobei die Volumina der ersten und zweiten Lösung ausreichend sind, um die F2-Lymphozyten von anderen peripheren Blutymphozyten zu trennen;

(b) Aufschichten eines Volumens peripherer Blutlymphozyten auf die geschichtete erste und zweite Lösung;

(c) Zentrifugieren der auf die geschichtete erste und zweite Lösung geschichteten peripheren Blutlymphozyten, so daß die F2-Lymphozyten-Subpopulation in einer sichtbaren Bande zwischen der geschichteten ersten und zweiten Lösung konzentriert ist; und

(d) Sammeln der sichtbaren Bande, um die F2-Lymphozyten-Subpopulation zu erhalten, wobei die gesammelten Lymphozyten im wesentlichen frei von Lymphozyten sind, die Schwebedichten von mehr als etwa 1,0670 $g/cm^3$ oder weniger als etwa 1,0590 $g/cm^3$ besitzen, wenn die Schwebedichten bei 20°C in einer Lösung mit einer Osmolalität von etwa 0,315 Osm/kg gemessen werden.

2. Verfahren zur Isolierung von sowohl F2- als auch F4-Subpopulationen von SCM-responsiven Lymphozyten aus der aus Blut isolierten Gesamtpopulation an peripheren Blutlymphozyten, folgende Schritte umfassend:

(a) Aufschichten einer ersten, zweiten, dritten und vierten Lösung mit der gleichen Osmolalität übereinander, um einen Lösungsstapel mit 4 Dichten zu bilden,

(i) wobei die erste Lösung eine Osmolalität zwischen etwa 0,270 Osm/kg und etwa 0,330 Osm/kg bei 20°C und einer Dichte ($\rho_1$), welche durch die Gleichung $\rho_1 = 1,0712 + 0,063$ (X - 0,290), worin X der Osmolalität der ersten Lösung in Osm/kg entspricht, bestimmt ist, besitzt;

(ii) die zweite Lösung eine Dichte ($\rho_2$) besitzt, die durch die Gleichung $\rho_2 = 1,0672 + 0,063$ (X - 0,290) bestimmt ist;

(iii) die dritte Lösung eine Dichte ($\rho_3$) besitzt, die durch die Gleichung $\rho_3 = 1,0652 + 0,063$ (X - 0,290) bestimmt ist;

(iii) die vierte Lösung eine Dichte ($\rho_4$) besitzt, die durch die Gleichung $\rho_4 = 1,0572 \div 0,063$ (X - 0,290) betimmt ist;

wobei die erste Lösung sich am Boden des Lösungsstapels befindet und die Volumina der vier Lösungen ausreichend sind, um die F2- und F4-Lymphozyten voneinander und von anderen peripheren Blutlymphozyten zu trennen;

(b) Aufschichten eines Volumens peripherer Blutlymphozyten auf den Lösungsstapel;

(c) Zentrifugieren der auf den Stapel mit 4 Dichten geschichteten peripheren Blutlymphozyten, so daß die F2-Lymphozyten-Subpopulation in einer sichtbaren Bande zwischen der dritten und vierten Lösung des Lösungsstapels konzentriert ist und die F4-Lymphozyten-Subpopulation in einer sichtbaren Bande zwischen der ersten und zweiten Lösung des Lösungsstapels konzentriert ist;

(d) Sammeln der sichtbaren Bande zwischen der dritten und vierten Lösung, um die F2-Lymphozyten-Subpopulation zu erhalten, wobei die gesammelten Lymphozyten im wesentlichen frei von Lymphozyten sind, die Schwebedichten von mehr als etwa 1,0670 g/cm$^3$ oder weniger als etwa 1,0590 g/cm$^3$ besitzen, wenn die Schwebedichten bei 20°C in einer Lösung mit einer Osmolalität von etwa 0,315 Osm/kg gemessen werden; und

(e) Sammeln der sichtbaren Bande zwischen der ersten und zweiten Lösung, um die F4-Lymphozyten-Subpopulation zu erhalten, wobei die gesammelten Lymphozyten im wesentlichen frei von Lymphozyten sind, die Schwebedichten von mehr als etwa 1,0730 g/cm$^2$ oder weniger als etwa 1,0690 g/cm$^3$ besitzen, wenn die Schwebedichten bei 20°C in einer Lösung mit einer Osmolalität von etwa 0,315 Osm/kg gemessen werden.

3. Verfahren zur Isolierung der F4-Subpopulation von SCM-responsiven Lymphozyten aus der aus Blut isolierten Gesamtpopulation an peripheren Blutlymphozyten, folgende Schritte umfassend:

(a) Aufschichten einer ersten Lösung mit einer Osmolalität zwischen etwa 0,270 Osm/kg und etwa 0,330 Osm/kg bei 20°C und einer Dichte ($\rho_1$), welche durch die Gleichung $\rho_1 = 1,0672 + 0,063$ (X - 0,290), worin X der Osmolalität der ersten Lösung in Osm/kg entspricht, bestimmt ist, auf eine zweite Lösung mit der gleichen Osmolalität wie die erste Lösung und einer Dichte ($\rho_2$), die durch die Gleichung $\rho_2 = 1,0712 + 0,063$ (X - 0,290) bestimmt ist, wobei die Volumina der ersten und zweiten Lösung ausreichend sind, um die F4-Lymphozyten von anderen peripheren Blutlymphozyten zu trennen;

(b) aufschichten eines Volumens peripherer Blutlymphozyten auf die geschichtete erste und zweite Lösung;

(c) Zentrifugieren der auf die geschichtete erste und zweite Lösung geschichteten peripheren Blutlymphozyten, so daß die F4-Lymphozyten-Subpopulation in einer sichtbaren Bande zwischen der geschichteten ersten und zweiten Lösung konzentriert ist; und

(d) Sammeln der sichtbaren Bande, um die F4-Lymphozyten-Subpopulation zu erhalten, wobei die gesammelten Lymphozyten im wesentlichen frei von Lymphozyten sind, die Schwebedichten von mehr als etwa 1,0730

g/cm$^3$ oder weniger als etwa 1,0690 g/cm$^3$ besitzen, wenn die *Schwebedichten* bei 20°C in einer Lösung mit einer Osmolalität von etwa 0,315 Osm/kg gemessen werden.

4. Verfahren nach Anspruch 1, wobei die Osmolalität der ersten und zweiten Lösung 0,320 Osm/kg beträgt, die erste Lösung eine Dichte von 1,0590 g/cm$^3$ und die zweite Lösung eine Dichte von 1,0670 g/cm$^3$ besitzt.

5. Verfahren nach Anspruch 2, wobei die Osmolalität der den Lösungsstapel mit vier Dichten bildenden Lösungen 0,320 Osm/kg beträgt, die erste Lösung eine Dichte von 1,0730 g/cm$^3$, die zweite Lösung eine Dichte von 1,0690 g/cm$^3$, die dritte Lösung eine Dichte von 1,0670 g/cm$^3$ und die vierte Lösung eine Dichte von 1,0590 g/cm$^3$ besitzt.

6. Verfahren nach Anspruch 3, wobei die Osmolalität der ersten und zweiten Lösung 0,320 Osm/kg aufweist, die erste Lösung eine Dichte von 1,0690 g/cm$^3$ und die zweite Lösung eine Dichte von 1,0730 g/cm$^3$ besitzt.

7. Vefahren nach Anspruch 1 oder 3, wobei die erste und zweite Lösung Lösungen von mit Polyvinylpyrrolidon bedecktem, kolloidalem Siliciumdioxid sind.

8. Verfahren nach Anspruch 2, wobei die vier Lösungen Lösungen von mit Polyvinylpyrrolidon bedecktern, kolloidalem Siliciumdioxid sind.

9. Verfahren nach Anspruch 1, 2 oder 3, ferner den Schritt des Isolierens der Gesamtpopulation von peripheren Blutlymphocyten aus einer heparinisierten Blutprobe durch Dichtegradientenzentrifugation in einem Medium umfassend, das zur Abtrennung der peripheren Blutlymphozyten von anderen Blutkomponenten in der Lage ist.

10. Verfahren nach Anspruch 9, worin der Schritt des Isolierens der Gesamtpopulation an peripheren Blutlymphozyten folgende Schritte umfaßt:

(i) Aufschichten von heparinisiertern Blut auf eine Lösung einer Dichte von 1,077 g/cm$^3$, die ein nichtionisches, synthetisches Polymer aus Saccharose mit einem Molekulargewicht von etwa 400 000 und Natriumdiatrizoat enthält, wobei das Volumen der Lösung der Dichte 1,077 g/cm$^3$ mindestens genauso groß wie das Volumen des heparinisierten Blutes ist und beide Lösungen auf Raumtemperatur äquilibriert sind,

(ii) Zentrifugieren der geschichteten Lösungen bei Raumtemperatur bei ausreichend wirksamer Gravitationskraft und für eine ausreichende Zeitspanne, so daß die Lymphozyten bandenförmig an der Grenzfläche zwischen der Lösung der Dichte von 1,077 g/cm$^3$ und dem heparinisiertem Blut vorliegen; und

(iii) Sammeln der Lymphozyten von der Grenzfläche.

11. Verfahren zum Testen einer Blutprobe auf die Anwesenheit eines Zustandes oder einer Krankheit, umfassend folgende Schritte:

(a) Heparinisieren der Blutprobe;

(b) Isolieren im wesentlichen aller peripherer Blutlymphozyten aus der heparinisierten Blutprobe;

(c) Getrenntes Isolieren der F2- als auch der F4-Subpopulation von Lymphozyten aus der Gesamtpopulation peripherer Blutlymphozyten durch folgende Schritte:

(i) Aufschichten einer ersten, zweiten, dritten und vierten Lösung mit der gleichen Osmolalität übereinander, um einen Lösungsstapel mit 4 Dichten zu bilden,

(a) wobei die erste Lösung eine Osmolalität zwischen etwa 0,270 Osm/kg und etwa 0,330 Osm/kg bei 20°C und einer Dichte ($\rho_1$), welche durch die Gleichung $\rho_1 = 1,0712 + 0,063 (X - 0,290)$, worin X der Osmolalitat der ersten Lösung in Osm/kg entspricht, bestimmt ist, besitzt;

(b) die zweite Lösung eine Dichte ($\rho_2$) besitzt, die durch die Gleichung $\rho_2 = 1,0672 + 0,063 (X - 0,290)$ bestimmt ist;

(c) die dritte Lösung eine Dichte ($\rho_3$) besitzt, die durch die Gleichung $\rho_3 - 1,0652 + 0,063 (X - 0,290)$

bestimmt ist;

(d) die vierte Lösung eine Dichte ($\rho_4$) besitzt, die durch die Gleichung $\rho_4 = 1{,}0572 + 0{,}063\,(X - 0{,}290)$ bestimmt ist;

wobei die erste Lösung sich am Boden des Lösungsstapels befindet und die Volumina der vier Lösungen ausreichend sind, um die F2- und F4-Lymphozyten voneinander und von anderen peripheren Blutlymphozyten zu trennen;

(ii) Aufschichten eines Volumens peripherer Blutlymphozyten auf den Lösungsstapel;

(iii) Zentrifugieren der auf den Stapel mit 4 Dichten geschichteten peripheren Blutlymphozyten, so daß die F2-Lymphozyten-Subpopulation in einer sichtbaren Bande zwischen der dritten und vierten Lösung des Lösungsstapels konzentriert ist und die F4-Lyitnphozyten-Subpopulation in einer sichtbaren Bande zwischen der ersten und zweiten Lösung des Lösungsstapels konzentriert ist;

(iv) Sammeln der sichtbaren Bande zwischen der dritten und vierten Lösung, um die F2-Lymphozyten-Subpopulation zu erhalten, wobei die gesammelten Lymphozyten im wesentlichen frei von Lymphozyten sind, die Schwebedichten von mehr als etwa 1,0670 g/cm$^3$ oder weniger als etwa 1,0590 g/cm$^3$ besitzen, wenn die Schwebedichten bei 20°C in einer Lösung mit einer Osmolalität von etwa 0,315 Osm/kg gemessen werden; und

(v) Sammeln der sichtbaren Bande zwischen der ersten und zweiten Lösung, um die F4-Lymphozyten-Subpopulation zu erhalten, wobei die gesammelten Lymphozyten im wesentlichen frei von Lymphozyten sind, die Schwebedichten von mehr als etwa 1,0730 g/cm$^3$ oder weniger als etwa 1,0690 g/cm$^3$ besitzen, wenn die Schwebedichten bei 20°C in einer Lösung mit einer Osmolalität von etwa 0,315 Osm/kg gemessen werden.

(d) Waschen der isolierten Subpopulationen der Lymphozyten;

(e) Hinzusetzen eines fluorogenen Präkursormittels zu jeder gewaschenen, isolierten Lymphozyten-Subpopulation;

(f) Kontaktieren eines Aliquots der gewaschenen F2-Lymphozyten-Subpopulation, der das fluorogene Präkursormittel hinzugesetzt worden ist, mit einer Substanz, die mit dem zu testenden Zustand oder der zu testenden Krankheit in Verbindung steht;

(g) Kontaktieren der gewaschenen F4-Lymphozyten-Subpopulation, der das fluorogene Präkursormittel hinzugesetzt worden ist, mit einem Mitogen, das zur Stimulierung von T-Zell-Lymphozyten in der Lage ist;

(h) Überprüfen der Response des Aliquots der mit der Substanz kontaktierten F2-Lymphozyten-Subpopulation, indem die Änderungen in der Strukturiertheit der cytoplasmatischen Matrix der Lymphozyten des Aliquots als ein Ergebnis des Kontaktes zwischen den Lymphozyten und der Substanz festgestellt werden;

(i) Überprüfen der Response der mit dem Mitogen kontaktierten F4-Lymphozyten-Subpopulation, indem die Änderungen in der Strukturiertheit der cytoplasmatischen Matrix der Lymphozyten als ein Ergebnis des Kontaktes zwischen den Lymphozyten und dem Mitogen festgestellt werden; und

(j) In-Bezug-Setzen der überprüften Response des Aliquots der F2-Lymphozyten-Sub-population und der F4-Lymphozyten-Subpopulation zum Vorhandensein oder zur Abwesenheit der Krankheit oder des Körperzustandes.

12. Verfahren zum Testen einer Blutprobe auf das Vorhandenseins eines Zustandes oder einer Krankheit, umfassend folgende Schritte:

(a) Heparinisieren der Blutprobe;

(b) Isolieren im wesentlichen aller peripherer Blutlymphozyten aus der heparinisierten Blutprobe;

(c) Isolieren der F2-Subpopulationen von Lymphozyten aus der Gesamtpopulation peripherer Blutlymphozyten durch folgende Schritte:

(i) Aufschichten einer ersten Lösung mit einer Osmolalität zwischen etwa 0,270 Osm/kg und etwa 0,330 Osm/kg bei 20°C und einer Dichte ($\rho_1$), welche durch die Gleichung $\rho_1 = 1,0572 + 0,063$ (X - 0,290), worin X der Osmolalität der ersten Lösung in Osm/kg entspricht, bestimmt ist, auf eine zweite Lösung mit der gleichen Osmolalität wie die erste Lösung und einer Dichte ($\rho_2$), die durch die Gleichung $\rho_2 = 1,0652 + 0,063$ (X - 0,290) bestimmt ist; wobei die Volumina der ersten und zweiten Lösung ausreichend sind, um die F2-Lymphozyter von anderen peripheren Blutlymphozyten zu trennen;

(ii) Aufschichten eines Volumens peripherer Blutlymphozyten auf die geschichtete erste und zweite Lösung;

(iii) Zentrifugieren der auf die geschichtete erste und zweite Lösung geschichteten peripheren Blutlymphozyten, so daß die F2-Lymphozyten-Subpopulation in einer sichtbaren Bande zwischen der geschichteten ersten und zweiten Lösung konzentriert ist; und

(iv) Sammeln der sichtbaren Bande, um die F2-Lymphozyten-Subpopulation zu erhalten, wobei die gesammelten Lymphozyten im wesentlichen frei von Lymphozyten sind, die Schwebedichten von mehr als etwa 1,0670 g/cm$^3$ oder weniger als etwa 1,0590 g/cm$^3$ besitzen, wenn die Schwebedichten bei 20°C in einer Lösung mit einer Osmolalität von etwa 0,315 Osm/kg gemessen werden;

(d) Waschen der isolierten F2-Subpopulation der Lymphozyten;

(e) Hinzusetzen eines fluorogenen Präkursormittels zu der gewaschenen, isolierten F2-Lymphozyten-Subpopulation;

(f) Kontaktieren eines Aliquots der gewaschenen F2-Lymphozyten-Subpopulation, der das fluorogene Präkursormittel hinzugesetzt worden ist, mit einer Substanz, die mit dem zu testenden Zustand oder der zu testenden Krankheit in Verbindung steht;

(g) Überprüfen der Response des Aliquots der mit der Substanz kontaktierten F2-Lymphozyten-Subpopulation, indem die Änderungen in der Strukturiertheit der cytoplasmatischen Matrix der Lymphozyten des Aliquots als ein Ergebnis des Kontaktes zwischen den Lymphozyten und der Substanz festgestellt werden;

(h) In-Bezug-Setzen der überprüften Response des Aliquots der F2-Lymphozyten-Subpopulation zum Vorhandensein oder zur Abwesenheit der Krankheit oder des Körperzustandes.

13. Verfahren zum Testen einer Blutprobe von einem Donor auf das Vorhandensein von Krebs beim Donor, umfassend folgende Schritte:

(a) Heparinisieren der Blutprobe;

(b) Isolieren im wesentlichen aller peripherer Blutlymphozyten aus der heparinisierten Blutprobe;

(c) Isolieren der F4-Subpopulationen von Lymphozyten aus der Gesamtpopulation peripherer Blutlymphozyten durch folgende Schritte:

(i) Aufschichten einer ersten Lösung mit einer Osmolalität zwischen etwa 0,270 Osm/kg und etwa 0,330 Osm/kg bei 20°C und einer Dichte ($\rho_1$), welche durch die Gleichung $\rho_1 = 1,0672 + 0,063$ (X - 0,290), worin X der Osmolalität der ersten Lösung in Osm/kg entspricht, bestimmt ist, auf eine zweite Lösung mit der gleichen Osmolalität wie die erste Lösung und einer Dichte ($\rho_2$), die durch die Gleichung $\rho_2 = 1,0712 + 0,063$ (X - 0,290) bestimmt ist; wobei die Volumina der ersten und zweiten Lösung ausreichend sind, um die F4-Lymphozyten von anderen peripheren Blutlymphozyten zu trennen;

(ii) Aufschichten eines Volumens peripherer Blutlymphozyten auf die geschichtete erste und zweite Lösung;

(iii) Zentrifugieren der auf die geschichtete erste und zweite Lösung geschichteten peripheren Blutlymphozyten, so daß die F4-Lymphozyten-Subpopulation in einer sichtbaren Bande zwischen der geschichteten ersten und zweiten Lösung konzentriert ist; und

(iv) Sammeln der sichtbaren Bande, um die F4-Lymphozyten-Subpopulation zu erhalten, wobei die gesammelten Lymphozyten im wesentlichen frei von Lymphozyten sind, die Schwebedichten von mehr als etwa $1{,}0730\ \text{g/cm}^3$ oder weniger als etwa $1{,}0690\ \text{g/cm}^3$ besitzen, wenn die Schwebedichten bei 20°C in einer Lösung mit einer Osmolalität von etwa 0,315 Osm/kg gemessen werden;

(d) Waschen der isolierten F4-Subpopulation der Lymphozyten;

(e) Hinzusetzen eines fluorogenen Präkursormittels zu der gewaschenen, isolierten F4-Lymphozyten- Subpopulation;

(f) Kontaktieren der gewaschenen F4-Lymphozyten-Subpopulation, der das fluorogene Präkursormittel hinzugesetzt worden ist, mit dem Mitogen;

(g) Überprüfen der Response der mit dem Mitogen kontaktierten F4-Lymphozyten-Subpopulation, indem die Änderungen in der Strukturiertheit der cytoplasmatischen Matrix der Lymphozyten des Aliquots als ein Ergebnis des Kontaktes zwischen den Lymphozyten und dem Mitogen festgestellt werden; und

(h) In-Bezug-Setzen der überprüften Response der F4-Lymphozyten-Subpopulation zum Vorhandensein oder zur Abwesenheit von Krebs.

14. Verfahren nach Anspruch 11 oder Anspruch 12, wobei der mit der Substanz in Verbindung stehende Zustand oder die mit der Substanz in Verbindung stehende Krankheit eine mit Krebs in Verbindung stehende Substanz ist.

15. Verfahren nach Anspruch 11, wobei die mit dem Zustand oder der Krankheit in Verbindung stehende Substanz aus der Gruppe gewählt wird, welche aus Virusassoziierten Antigenen und Bakterien-assoziierten Antigenen besteht.

16. Verfahren nach Anspruch 11, wobei die mit dem Zustand oder der Krankheit in Verbindung stehende Substanz in der Lage ist, eine Abnahme in der Strukturiertheit der cytoplasmatischen Matrix von Lymphozyten hervorzurufen, die inkompatibel mit dem Donor der die Substanz produzierenden Lymphozyten sind, und als ein Indikator der Allotransplantat-Abstoβungskrise vorliegt.

17. Verfahren nach Anspruch 11, wobei die Substanz Gewebeextrakt aus einem Transplantat-Donor ist und der Schritt des In-Bezug-Setzens der überprüften Response das Bestimmen der Kompatibilität für eine Organtransplantation zwischen Transplantat-Empfanger, aus dem die Blutprobe erhalten wurde, und dem Transplantat-Donor, aus dem der Gewebeextrakt erhalten wurde, umfaßt.

18. Verfahren nach Anspruch 11, umfassend die zusätzlichen Schritte:

(a) Kontaktieren auch eines zweiten Aliquots der gewaschenen F2-Lymphozyten-Subpopulation, der das fluorogene Präkursormittel hinzugesetzt worden ist, mit dem Mitogen, das zur Stimulierung von T-Zell-Lymphozyten in der Lage ist;

(b) Überprüfen der Response des mit dem Mitogen kontaktierten zweiten Aliquots der F2-Lymphozyten-subpopulation, indem die Änderungen in der Strukturiertheit der cytoplasmatischen Matrix der Lymphozyten des Aliquots als ein Ergebnis des Kontaktes zwischen den Lymphozyten und dem Mitogen festgestellt werden; und

(c) In-Bezug-Setzen der überprüften Response des zweiten Aliquots der F2-Lymphozyten-Subpopulation zum Vorhandensein oder zur Abwesenheit der Krankheit oder des Körperzustandes, um die mit dem ersten Aliquot der F2-Lymphozyten-Subpopulation erhaltenen Ergebnisse zu bestätigen.

19. Verfahren nach Anspruch 12, umfassend die zusätzlichen Schritte:

(a) Kontaktieren auch eines zweiten Aliquots der gewaschenen F2-Lymphozyten-Subpopulation, der das fluorogene Präkursomittel hinzugesetzt worden ist, mit einem Mitogen, das zur Stimulierung von T-Zell-Lymphozyten in der Lage ist;

(b) Überprüfen der Response des mit dem Mitogen kontaktierten zweiten Aliquots der F2-Lymphozyten-Subpopulation, indem die Änderungen in der Strukturiertheit der cytoplasmatischen Matrix der Lymphozyten des zweiten Aliquots als ein Ergebnis des Kontaktes zwischen den Lymphozyten und dem Mitogen festgestellt werden; und

(c) In-Bezug-Setzen der überprüften Response des zweiten Aliquots der F2-Lymphozyten-Subpopulation zum Vorhandensein oder zur Abwesenheit der Krankheit oder des Körperzustandes, um die mit dem ersten Aliquot der F2-Lymphozyten-Subpopulation und mit der F4-Lymphozyten-Subpopulation erhaltenen Ergebnisse zu bestätigen

20. Verfahren nach Anspruch 11, 13, 18 oder 19, wobei das Mitogen analysenreines Phytohämagglutinin ist.

21. Verfahren nach Anspruch 11, wobei die Osmolalität der den Lösungsstapel mit vier Dichten bildenden Lösungen 0,320 Osm/kg beträgt, die erste Lösung eine Dichte von 1,0730 g/cm$^3$, die zweite Lösung eine Dichte von 1,0690 g/cm$^3$, die dritte Lösung eine Dichte von 1,0670 g/cm$^3$ und die vierte Lösung eine Dichte von 1,0590 g/cm$^3$ besitzt.

22. Verfahren nach Anspruch 12, wobei die Osmolalität der ersten und zweiten Lösung 0,320 Osm/kg beträgt, die erste Lösung eine Dichte von 1,0590 g/cm$^3$ und die zweite Lösung eine Dichte von 1,0670 g/cm$^3$ besitzt.

23. Verfahren nach Anspruch 13, wobei die Osmolalität der ersten und zweiten Lösung 0,320 Osm/kg beträgt, die erste Lösung eine Dichte von 1,0690 g/cm$^3$ und die zweite Lösung eine Dichte von 1,0730 g/cm$^3$ besitzt.

24. Verfahren nach Anspruch 11, 12 oder 13, wobei der Schritt des isolierens im wesentlichen aller peripherer Blutlymphozyten aus der heparinisierten Blutprobe folgende Schritte umfaßt:

(i) Aufschichten von heparinisiertem Blut auf eine Lösung einer Dichte von 1,077 g/cm$^3$, die ein nichtionisches, synthetisches Polymer aus Saccharose mit einem Molekulargewicht von etwa 400 000 und Natriumdiatrizoat enthält, wobei das Volumen der Lösung der Dichte 1,077 g/cm$^3$ mindestens genauso groß wie das Volumen des heparinisierten Blutes ist und beide Lösungen auf Raumtemperatur äquilibriert sind;

(ii) Zentrifugieren der geschichteten Lösungen bei Raumtemperatur bei ausreichend wirksamer Gravitationskraft und für eine ausreichende Zeitspanne, so daß die Lymphozyten banderformig an der Grenzfläche zwischen der Lösung der Dichte von 1,077 g/cm$^3$ und dem heparinisiertem Blut vorliegen; und

(iii) Sammeln der Lymphozyten aus der Grenzfläche.

25. Verfahren nach Anspruch 11, wobei die vier Lösungen Lösungen von mit Polyvinylpyrrolidon bedecktem, kolloidalem Siliciumdioxid sind.

26. Verfahren nach Anspruch 12 oder 13, wobei die erste und zweite Lösung Lösungen von mit Polyvinylpyrrolidon bedecktem, kolloidalem Siliciumdioxid sind.

## Revendications

1. Méthode d'isolement de la sous-population F2 de lymphocytes répondant à la SCM (structuration de la matrice cytoplasmique) à partir de la population totale de lymphocytes du sang périphérique isolés à partir de sang comprenant les étapes consistant à :

(a) disposer en couche une première solution ayant une osmolalité comprise entre environ 0,270 Osm/kg et environ 0,330 Osm/kg à 20°C et une densité ($\rho_1$) déterminée par l'équation $\rho_1 = 1,0572 + 0,063 (X - 0,290)$, où X est égal à l'osmolalité de la première solution en Osm/kg, par dessus une deuxième solution avec la même osmolalité que la première solution et une densité ($\rho_2$) déterminée par l'équation $\rho_2 = 1,0652 + 0,063 (X - 0,290)$, les volumes des première et deuxième solutions étant suffisants pour séparer les lymphocytes F2 des autres lymphocytes du sang périphérique ;

(b) disposer en couche un volume de lymphocytes du sang périphérique par dessus les première et deuxième solutions disposées en couches ;

(c) centrifuger les lymphocytes du sang périphérique disposés en couche par dessus les première et deuxième solutions disposées en couche de façon que la sous-population de lymphocytes F2 soit concentrée en une bande visible entre les première et deuxième solutions disposées en couches ; et

(d) recueillir la bande visible pour obtenir la sous-population de lymphocytes F2, les lymphocytes recueillies étant sensiblement exempts de lymphocytes ayant des densités à l'équilibre supérieures à environ 1,0670 g/cm$^3$ ou inférieures à environ 1,0590 g/cm$^3$, les densités à l'équilibre étant mesurées à 20°C dans une solution ayant une osmolalité d'environ 0,315 Osm/kg.

2.  Méthode d'isolement à la fois des sous-populations F2 et F4 de lymphocytes répondant à la SCM à partir de la population totale de lymphocytes du sang périphérique isolés à partir de sang comprenant les étapes consistant à :

   (a) disposer en couche, une première, deuxième, troisième et quatrième solution ayant la même osmolalité, l'une par dessus l'autre, pour former une superposition de solutions à quatre densités :

   (i) la première solution ayant une osmolalité comprise entre environ 0,270 Osm/kg et environ 0,330 Osm/kg à 20°C et une densité ($\rho_1$) déterminée par l'équation $\rho_1 = 1,0712 + 0,063 \, (X - 0,290)$, où X est égal à l'osmolalité de la première solution en Osm/kg ;

   (ii) la deuxième solution ayant une densité ($\rho_2$) déterminée par l'équation $\rho_2 = 1,0672 + 0,063 \, (X - 0,290)$ ;

   (iii) la troisième solution ayant une densité ($\rho_3$) déterminée par l'équation $\rho_3 = 1,0652 + 0,063 \, (X - 0,290)$ ;

   (iv) la quatrième solution ayant une densité ($\rho_4$) déterminée par l'équation $\rho_4 = 1,0572 + 0,063 \, (X - 0,290)$ ; la première solution étant positionnée au fond de la superposition de solutions, les volumes des quatre solutions étant suffisants pour séparer les lymphocytes F2 et F4 les uns des autres ainsi que des autres lymphocytes du sang périphérique ;

   (b) disposer en couche un volume de lymphocytes du sang périphérique au sommet de la superposition de solutions ;

   (c) centrifuger les lymphocytes du sang périphérique disposés en couche par dessus la superposition de solutions à quatre densités de façon que la sous-population de lymphocytes F2 soit concentrée en une bande visible entre les troisième et quatrième solutions de la superposition de solutions et la sous-population de lymphocytes F4 soit concentrée en une bande visible entre les première et deuxième solutions de la superposition de solutions ;

   (d) recueillir la bande visible entre les troisième et quatrième solutions pour obtenir la sous-population de lymphocytes F2, les lymphocytes recueillis étant sensiblement exempts de lymphocytes ayant des densités à l'équilibre supérieures à environ 1,0670 g/cm$^3$ ou inférieures à environ 1,0590 g/cm$^3$, les densités à l'équilibre étant mesurées à 20°C dans une solution ayant une osmolalité d'environ 0,315 Osm/kg ; et

   (e) recueillir la bande visible entre les première et deuxième solutions pour obtenir la sous-population de lymphocytes F4, les lymphocytes recueillis étant sensiblement exempts de lymphocytes ayant des densités à l'équilibre supérieures à environ 1,0730 g/cm$^3$ ou inférieures à environ 1,0690 g/cm$^3$, les densités à l'équilibre étant mesurées à 20°C dans une solution ayant une osmolalité d'environ 0,315 Osm/kg.

3.  Méthode d'isolement de la sous-population F4 de lymphocytes répondant à la SCM à partir de la population totale de lymphocytes du sang périphérique isolés à partir de sang comprenant les étapes consistant à :

   (a) disposer en couche une première solution ayant une osmolalité comprise entre environ 0,270 Osm/kg et environ 0,330 Osm/kg à 20°C et une densité ($\rho_1$) déterminée par l'équation $\rho_1 = 1,0672 + 0,063 \, (X - 0,290)$, où X est égal à l'osmolalité de la première solution en Osm/kg, par dessus une deuxième solution ayant la même osmolalité que la première solution et une densité ($\rho_2$) déterminée par l'équation $\rho_2 = 1,0712 + 0,063 \, (X - 0,290)$, les volumes des première et deuxième solutions étant suffisants pour séparer les lymphocytes F4 des autres lymphocytes du sang périphérique ;

   (b) disposer en couche un volume de lymphocytes du sang périphérique par dessus les première et deuxième solutions disposées en couche;

(c) centrifuger les lymphocytes du sang périphérique disposés en couche par dessus les première et deuxième solutions disposées en couche de façon que la sous-population de lymphocytes F4 soit concentrée en une bande visible entre les première et deuxième solutions disposées en couche; et

(d) recueillir la bande visible pour obtenir la sous-population de lymphocytes F4, les lymphocytes recueillis étant sensiblement exempts de lymphocytes ayant des densités à l'équilibre supérieures à environ 1,0730 g/cm$^3$ ou inférieures à environ 1,0690 g/cm$^3$, les densités à l'équilibre étant mesurées à 20°C dans une solution ayant une osmolalité d'environ 0,315 Osm/kg.

4. Méthode de la revendication 1 dans laquelle l'osmolalité des première et deuxième solutions est de 0,320 Osm/kg, la première solution a une densité de 1,0590 g/cm$^3$, et la deuxième solution a une densité de 1,0670 g/cm$^3$.

5. Méthode selon la revendication 2 dans laquelle l'osmolalité des solutions formant la superposition de solutions à quatre densités est de 0,320 Osm/kg, la première solution a une densité de 1,0730 g/cm$^3$, la deuxième solution a une densité de 1,0690 g/cm$^3$, la troisième solution a une densité de 1,0670 g/cm$^3$, et la quatrième solution a une densité de 1,0590 g/cm$^3$.

6. Méthode selon la revendication 3 dans laquelle l'osmolalité des première et deuxième solutions est de 0,320 Osm/kg, la première solution a une densité de 1,0690 g/cm$^3$, et la deuxième solution a une densité de 1,0730 g/cm$^3$.

7. Méthode selon la revendication 1 ou 3 dans laquelle les première et deuxième solutions sont des solutions de silice colloïdale recouverte de polyvinylpyrrolidone.

8. Méthode selon la revendication 2 dans laquelle les quatre solutions sont des solutions de silice colloïdale recouverte de polyvinylpyrrolidone.

9. Méthode de la revendication 1, 2, ou 3 comprenant en outre l'étape consistant à isoler la population totale de lymphocytes du sang périphérique à partir d'un échantillon de sang hépariné par une centrifugation en gradient de densité dans un milieu capable de séparer les lymphocytes du sang périphérique des autres composants du sang.

10. Méthode selon la revendication 9 dans laquelle l'étape d'isolement de la population totale des lymphocytes du sang périphérique comprend les étapes consistant à :

(i) disposer en couche du sang hépariné par dessus une solution de densité 1,077 g/cm$^3$ contenant un polymère synthétique non ionique de saccharose avec un poids moléculaire d'environ 400 000 et du diatrizoate de sodium, le volume de la solution de densité 1,077 g/cm$^3$ étant au moins aussi grand que le volume de sang hépariné, les deux solutions étant équilibrées à température ambiante ;

(ii) centrifuger les solutions disposées en couches à température ambiante avec une force de gravité suffisamment efficace et pendant un temps suffisant pour que les lymphocytes forment une bande à l'interface entre la solution de densité 1,077 g/cm$^3$ et le sang hépariné ; et

(iii) recueillir les lymphocytes à partir de l'interface.

11. Méthode pour rechercher dans un échantillon de sang la présence d'un état ou d'une maladie comprenant les étapes consistant à :

(a) hépariner l'échantillon de sang ;

(b) isoler sensiblement tous les lymphocytes du sang périphérique à partir de l'échantillon de sang hépariné ;

(c) isoler séparément à la fois les sous-populations de lymphocytes F2 et F4 à partir de la population totale de lymphocytes du sang périphérique par les étapes consistant à :

(i) disposer en couche une première, deuxième, troisième, et quatrième solution avec la même osmolalité l'une par dessus l'autre pour former une superposition de solutions à quatre densités :

[a] la première solution ayant une osmolalité comprise entre environ 0,270 Osm/kg et environ 0,330 Osm/kg à 20°C et une densité ($\rho_1$) déterminée par l'équation $\rho_1 = 1,0712 + 0,063\ (X - 0,290)$, où X est égal à l'osmolalité de la première solution en Osm/kg ;

[b] la deuxième solution ayant une densité ($\rho_2$) déterminée par l'équation $\rho_2 = 1,0672 + 0,063\ (X - 0,290)$ ;

[c] la troisième solution ayant une densité ($\rho_3$) déterminée par l'équation $\rho_3 = 1,0652 + 0,063\ (X - 0,290)$ ; et

[d] la quatrième solution ayant une densité ($\rho_4$) déterminée par l'équation $\rho_4 = 1,0572 + 0,063\ (X - 0,290)$ ;

la première solution étant positionnée au fond de la superposition de solutions, les volumes des quatre solutions étant suffisants pour séparer les lymphocytes F2 et F4 les uns des autres ainsi que des autres lymphocytes du sang périphérique ;

(ii) disposer en couche un volume de lymphocytes de sang périphérique au sommet de la superposition de solutions ;

(iii) centrifuger les lymphocytes du sang périphérique disposés en couche par dessus la superposition de solutions à quatre densités de façon que la sous-population de lymphocytes F2 soit concentrée en une bande visible entre les troisième et quatrième solutions de la superposition de solutions et la sous-population de lymphocytes F4 soit concentrée en une bande visible entre les première et deuxième solutions de la superposition de solutions ;

(iv) recueillir la bande visible entre les troisième et quatrième solutions pour obtenir la sous-population de lymphocytes F2, les lymphocytes recueillis étant sensiblement exempts de lymphocytes ayant des densités à l'équilibre supérieures à environ 1,0670 $g/cm^3$ ou inférieures à environ 1,0590 $g/cm^3$, les densités à l'équilibre étant mesurées à 20°C dans une solution ayant une osmolalité d'environ 0,315 Osm/kg ; et

(v) recueillir la bande visible entre les première et deuxième solutions pour obtenir la sous-population de lymphocytes F4, les lymphocytes recueillis étant sensiblement exempts de lymphocytes ayant des densités à l'équilibre supérieures à environ 1,0730 $g/cm3$ ou inférieures à environ 1,0690 $g/cm3$, les densités à l'équilibre étant mesurées à 20°C dans une solution ayant une osmolalité d'environ 0,315 Osm/kg ;

(d) laver les sous-populations isolées de lymphocytes;

(e) ajouter un précurseur d'agent fluorogène à chacune des sous-populations de lymphocytes isolées et lavées ;

(f) mettre en contact une fraction aliquote de la sous-population de lymphocytes F2 lavées à laquelle on a ajouté le précurseur d'agent fluorogène avec une substance associée à l'état ou maladie que l'on recherche;

(g) mettre en contact la sous-population de lymphocytes F4 lavées à laquelle le précurseur d'agent fluorogène a été ajouté avec un mitogène capable de stimuler les lymphocytes T ;

(h) suivre la réponse de la fraction aliquote de la sous-population de lymphocytes F2 mise en contact avec la substance en observant les changements de la structuration de la matrice cytoplasmique des lymphocytes de la fraction aliquote résultant du contact entre les lymphocytes et la substance ;

(i) suivre la réponse de la sous-population de lymphocytes F4 mise en contact avec le mitogène en observant les changements de la structuration de la matrice cytoplasmique des lymphocytes résultant du contact entre les lymphocytes et le mitogène ; et

(j) mettre en relation la réponse suivie de la fraction aliquote de la sous-population de lymphocytes F2 et de la sous-population de lymphocytes F4 et la présence ou l'absence de la maladie ou de l'état corporel.

12. Méthode pour rechercher dans un échantillon de sang la présence d'un état ou d'une maladie comprenant les étapes consistant à :

(a) hépariner l'échantillon de sang ;

(b) isoler sensiblement tous les lymphocytes du sang périphérique à partir de l'échantillon de sang hépariné ;

(c) isoler la sous-population F2 de lymphocytes à partir de la population totale de lymphocytes du sang périphérique par les étapes consistant à :

(i) disposer en couche une première solution ayant une osmolalité comprise entre environ 0,270 Osm/kg et environ 0,330 Osm/kg à 20°C et une densité ($\rho_1$) déterminée par l'équation $\rho_1 = 1,0572 + 0,063$ (X - 0,290), où X est égal à l'osmolalité de la première solution en Osm/kg par dessus une deuxième solution avec la même osmolalité que la première solution et une densité ($\rho_2$) déterminée par l'équation $\rho_2 = 1,0652 + 0,063$ (X - 0,290), les volumes des première et deuxième solutions étant suffisants pour séparer les lymphocytes F2 des autres lymphocytes du sang périphérique ;

(ii) disposer en couche un volume de lymphocytes du sang périphérique par dessus les première et deuxième solutions disposées en couche;

(iii) centrifuger les lymphocytes du sang périphérique disposés en couche par dessus les première et deuxième solutions disposées en couche de façon que la sous-population de lymphocytes F2 soit concentrée en une bande visible entre les première et deuxième solutions disposées en couche; et

(iv) recueillir la bande visible pour obtenir la sous-population de lymphocytes F2, les lymphocytes recueillis étant sensiblement exempts de lymphocytes ayant des densités à l'équilibre supérieures à environ 1,0670 g/cm$^3$ ou inférieures à environ 1,0590 g/cm$^3$, les densités à l'équilibre étant mesurées à 20°C dans une solution ayant une osmolalité d'environ 0,315 Osm/kg ;

(d) laver la sous-population isolée de lymphocytes F2;

(e) ajouter un précurseur d'agent fluorogène à la sous-population de lymphocytes F2 isolées et lavées ;

(f) mettre en contact une fraction aliquote de la sous-population de lymphocytes F2 lavée à laquelle on a ajouté le précurseur d'agent fluorogène avec une substance associée à l'état ou maladie que l'on recherche;

(g) suivre la réponse de la fraction aliquote de la sous-population de lymphocytes F2 mise en contact avec la substance en observant les changements de la structuration de la matrice cytoplasmique des lymphocytes de la fraction aliquote résultant du contact entre les lymphocytes et la substance ; et

(h) mettre en relation la réponse suivie de la fraction aliquote de la sous-population de lymphocytes F2 et la présence ou l'absence de la maladie ou de l'état corporel.

13. Méthode pour rechercher dans un échantillon de sang d'un donneur la présence d'un cancer chez le donneur comprenant les étapes consistant à :

(a) hépariner l'échantillon de sang ;

(b) isoler sensiblement tous les lymphocytes du sang périphérique à partir de l'échantillon de sang hépariné ;

(c) isoler la sous-population F4 des lymphocytes à partir de la population totale de lymphocytes du sang périphérique par les étapes consistant à :

(i) disposer en couche une première solution ayant une osmolalité comprise entre environ 0,270 Osm/kg et environ 0,330 Osm/kg à 20°C et une densité ($\rho_1$) déterminée par l'équation $\rho_1 = 1,0672 + 0,063$ (X - 0,290), où X est égal à l'osmolalité de la première solution en Osm/kg, par dessus une deuxième solution ayant la même osmolalité que la première solution et une densité ($\rho_2$) déterminée par l'équation $\rho_2 = 1,0712 + 0,063$ (X - 0,290), les volumes des première et deuxième solutions étant suffisants pour séparer les lymphocytes F4 des autres lymphocytes du sang périphérique ;

(ii) disposer en couche un volume de lymphocytes du sang périphérique par dessus les première et deuxième solutions disposées en couche;

(iii) centrifuger les lymphocytes du sang périphérique disposés en couche par dessus les première et deuxième solutions disposées en couche de façon que la sous-population de lymphocytes F4 soit concentrée en une bande visible entre les première et deuxième solutions disposées en couche; et

(iv) recueillir la bande visible pour obtenir la sous-population de lymphocytes F4, les lymphocytes recueillis étant sensiblement exempts de lymphocytes ayant des densités à l'équilibre supérieures à environ 1,0730 $g/cm^3$ ou inférieures à environ 1,0690 $g/cm^3$, les densités à l'équilibre étant mesurées à 20°C dans une solution ayant une osmolalité d'environ 0,315 Osm/kg ;

(d) laver la sous-population de lymphocytes F4 isolée ;

(e) ajouter un précurseur d'agent fluorogène à la sous-population de lymphocytes F4 isolée et lavée ;

(f) mettre en contact la sous-population de lymphocytes F4 lavée à laquelle on a ajouté le précurseur d'agent fluorogène avec le mitogène ;

(g) suivre la réponse de la sous-population de lymphocytes F4 mise en contact avec le mitogène en observant les changements de la structuration de la matrice cytoplasmique des lymphocytes de la fraction aliquote résultant du contact entre les lymphocytes et le mitogène ; et

(h) mettre en relation la réponse suivie de la sous-population de lymphocytes F4 avec la présence ou l'absence d'un cancer.

14. Méthode selon la revendication 11 ou la revendication 12 dans laquelle la substance associée à un état ou une maladie est une substance associée à un cancer.

15. Méthode selon la revendication 11 dans laquelle la substance associée à un état ou une maladie est choisie parmi le groupe consistant en des antigènes associés viraux et des antigènes associés bactériens.

16. Méthode selon la revendication 11 dans laquelle la substance associée à un état ou une maladie est capable de causer une diminution de la structuration de la matrice cytoplasmique des lymphocytes incompatibles avec le donneur des lymphocytes produisant la substance et est présente en tant qu'indicateur d'une crise de rejet d'une allogreffe.

17. Méthode selon la revendication 11 dans laquelle la substance est un extrait tissulaire à partir d'un donneur de transplant et l'étape de mise en relation de la réponse suivie comprend la détermination de la compatibilité pour une transplantation d'organes entre un receveur de transplant, à partir duquel l'échantillon de sang a été obtenu, et le donneur de transplant à partir duquel l'extrait tissulaire a été obtenu.

18. Méthode selon la revendication 11 comprenant les étapes supplémentaires consistant à :

(a) mettre également en contact une deuxième fraction aliquote de la sous-population de lymphocytes F2 lavée à laquelle le précurseur d'agent fluorogène a été ajouté avec le mitogène capable de stimuler les lymphocytes T ;

(b) suivre la réponse de la deuxième fraction aliquote mise en contact avec le mitogène de la sous-population de lymphocytes F2 en observant les changements de la structuration de la matrice cytoplasmique des lymphocytes de la fraction aliquote résultant de la mise en contact entre les lymphocytes et le mitogène ; et

(c) mettre en relation la réponse suivie de la deuxième fraction aliquote de la sous-population de lymphocytes F2 avec la présence ou l'absence de la maladie ou de l'état corporel pour confirmer le résultat obtenu avec la première fraction aliquote de la sous-population de lymphocytes F2.

19. Méthode selon la revendication 12 comprenant les étapes supplémentaires consistant à :

(a) mettre également en contact une deuxième fraction aliquote de la sous-population de lymphocytes F2 lavée à laquelle le précurseur d'agent fluorogène a été ajouté avec un mitogène capable de stimuler des lymphocytes T ;

(b) suivre la réponse vis-à-vis du mitogène de la deuxième fraction aliquote de la sous-population de lymphocytes F2 mise en contact avec le mitogène en observant les changements de la structuration de la matrice cytoplasmique des lymphocytes de la deuxième fraction aliquote résultant du contact entre les lymphocytes et le mitogène ; et

(c) mettre en relation la réponse suivie de la deuxième fraction aliquote de la sous-population de lymphocytes F2 avec la présence ou l'absence de la maladie ou de l'état corporel pour confirmer le résultat obtenu avec la première fraction aliquote de la sous-population de lymphocytes F2 et avec la sous-population de lymphocytes F4.

20. Méthode selon la revendication 11, 13, 18 ou 19 dans laquelle le mitogène est de la phytohémagglutinine de qualité appropriée comme réactif.

21. Méthode selon la revendication 11 dans laquelle l'osmolalité des solutions formant la superposition de solutions à quatre densités est de 0,320 Osm/kg, la première solution a une densité de 1,0730 g/cm$^3$, la deuxième solution a une densité de 1,0690 g/cm$^3$, la troisième solution a une densité de 1,0670 g/cm$^3$, et la quatrième solution a une densité de 1,0590 g/cm$^3$.

22. Méthode selon la revendication 12 dans laquelle l'osmolalité des première et deuxième solutions est de 0,320 Osm/kg, la première solution a une densité de 1,0590 g/cm$^3$, et la deuxième solution a une densité de 1,0670 g/cm$^3$.

23. Méthode selon la revendication 13 dans laquelle l'osmolalité des première et deuxième solutions est de 0,320 Osm/kg, la première solution a une densité de 1,0690 g/cm$^3$, et la deuxième solution a une densité de 1,0730 g/cm$^3$.

24. Méthode selon la revendication 11, 12 ou 13 dans laquelle l'étape consistant à isoler sensiblement tous les lymphocytes du sang périphérique à partir de l'échantillon de sang hépariné comprend les étapes consistant à :

(i) disposer en couche du sang hépariné par dessus une solution de densité 1,077 g/cm$^3$ contenant un polymère synthétique non ionique de saccharose avec un poids moléculaire d'environ 400 000 et du diatrizoate de sodium, le volume de la solution de densité 1,077 g/cm$^3$ étant au moins aussi grand que le volume de sang hépariné, les deux solutions étant équilibrées à température ambiante ;

(ii) centrifuger les solutions disposées en couches à température ambiante avec une force de gravité suffisamment efficace et pendant un temps suffisant pour que les lymphocytes forment une bande à l'interface entre la solution de densité 1,077 g/cm$^3$ et le sang hépariné ; et

(iii) recueillir les lymphocytes à partir de l'interface.

25. Méthode selon la revendication 11 dans laquelle les quatre solutions sont des solutions de silice colloïdale recouverte de polyvinylpyrrolidone.

26. Méthode selon la revendication 12 ou 13 dans laquelle les première et deuxième solutions sont des solutions de silice colloïdale recouverte de polyvinylpyrrolidone.

FIG.1.

Fig. 2.